# EUROPÄISCHE PATENTSCHRIFT

(11) **EP 2 432 892 B1**
(45) Veröffentlichungstag und Bekanntmachung des Hinweises auf die Patenterteilung: **08.06.2016**
(21) Anmeldenummer: 10722917.1
(22) Anmeldetag: 01.04.2010
(51) Int. Cl.: C12P 21/02, C12P 33/00, C07K 14/705, C12N 15/81, G01N 33/68

(54) **MIKROORGANISMUS ZUR EXPRESSION EINES HUMANEN MEMBRANPROTEINS**
MICROORGANISM FOR EXPRESSING A HUMAN MEMBRANE PROTEIN
MICRO-ORGANISME PERMETTANT L'EXPRESSION D'UNE PROTÉINE MEMBRANAIRE HUMAINE

(30) Priorität: 21.05.2009 DE 102009022772
(43) Veröffentlichungstag der Anmeldung: 28.03.2012
(73) Patentinhaber: OrganoBalance GmbH, 13355 Berlin (DE)
(72) Erfinder: SCHILLING, Michael, 10439 Berlin (DE); LANG, Christine, 14057 Berlin (DE); RAAB, Andreas, 13156 Berlin (DE)
(74) Vertreter: Jungblut, Bernhard Jakob
(86) Internationale Anmeldenummer: PCT/DE2010/000401
(87) Internationale Veröffentlichungsnummer: WO 2010/133194

(56) Entgegenhaltungen:
- EP-A2- 0 486 290
- WO-A1-03/064650
- US-A- 5 759 801
- DATABASE WPI Week 200436 Thomson Scientific, London, GB; AN 2004-382697 XP002596576 -& JP 2004 141125 A (MITSUBISHI CHEM CORP) 20. Mai 2004 (2004-05-20)
- TATE C G ET AL: "Comparison of seven different heterologous protein expression systems for the production of the serotonin transporter" BIOCHIMICA ET BIOPHYSICA ACTA, Bd. 1610, Nr. 1, 17. Februar 2003 (2003-02-17), Seiten 141-153, XP004409765 ISSN: 0005-2736 in der Anmeldung erwähnt
- SAMULI OLLILA ET AL: "Role of sterol type on lateral pressure profiles of lipid membranes affecting membrane protein functionality: Comparison between cholesterol, desmosterol, 7-dehydrocholesterol and ketosterol" JOURNAL OF STRUCTURAL BIOLOGY, Bd. 159, Nr. 2, 26. Juli 2007 (2007-07-26) , Seiten 311-323, XP022170688 ISSN: 1047-8477
- TATE C G: "Overexpression of mammalian integral membrane proteins for structural studies" FEBS LETTERS, Bd. 504, Nr. 3, 31. August 2001 (2001-08-31), Seiten 94-98, XP004597918 ISSN: 0014-5793
- OPEKAROVA M ET AL: "Specific lipid requirements of membrane proteins-a putative bottleneck in heterologous expression" BIOCHIMICA ET BIOPHYSICA ACTA. BIOMEMBRANES, Bd. 1610, Nr. 1, 17. Februar 2003 (2003-02-17), Seiten 11-22, XP004409752 ISSN: 0005-2736
- WAGNER S ET AL: "Rationalizing membrane protein overexpression" TRENDS IN BIOTECHNOLOGY, Bd. 24, Nr. 8, 1. August 2006 (2006-08-01) , Seiten 364-371, XP025052258 ISSN: 0167-7799 [gefunden am 2006-08-01]
- MIDGETT ET AL: "Breaking the bottleneck: Eukaryotic membrane protein expression for high-resolution structural studies" JOURNAL OF STRUCTURAL BIOLOGY, Bd. 160, Nr. 3, 15. November 2007 (2007-11-15), Seiten 265-274, XP022346076 ISSN: 1047-8477
- EIFLER ET AL: "Functional expression of mammalian receptors and membrane channels in different cells" JOURNAL OF STRUCTURAL BIOLOGY, Bd. 159, Nr. 2, 26. Juli 2007 (2007-07-26) , Seiten 179-193, XP022170675 ISSN: 1047-8477

## Beschreibung

### Gebiet der Erfindung

Die Erfindung betrifft einen isolierten genetisch veränderten lebenden nicht-Säuger-Organismus, mit einer gegenüber dem Wildtyp erhöhten HMG-CoA-Reduktase Aktivität, und mit einer gegenüber dem Wildtyp reduzierten C24-Methyltransferase- und/oder delta22-Desaturase-Aktivität, dessen Verwendungen, ein Nukleinsäurekonstrukt zu dessen Herstellung, ein Kit enthaltend den Organismus, sowie ein Membranextrakt, erhältlich aus dem Organismus.

Stand der Technik und Hintergrund der Erfindung.

Ein Organismus des eingangs genannten Aufbaus ist aus der Literaturstelle.WO 03/064650 bekannt. Dieser Organismus ist zur Biosynthese von 7-Dehydrocholesterol geeignet. Die Motivation zur Schaffung dieses Organismus beruht auf dem Bedarf, Vorprodukte zu Vitamin D3, insbesondere 7-Dehydrocholesterol, in großen Mengen und unter wirtschaftlichen Bedingungen herzustellen. Ähnliche Organismen sind aus den Literaturstellen Database WPI Week 200436 AN2004 - 382697, JP 2004141125 A und EP 0486290 A2 bekannt.

In völlig anderem Zusammenhang besteht ein Bedarf Membranproteine, insbesondere humane Membranproteine, funktionell in nativer Konförmation in zumindest analytisch gut brauchbaren Mengen herzustellen bzw. System zur Verfügung zu stellen, welche diese Proteine in einer beispielsweise für Screeningzwecke hinreichenden Menge funktionell aktiv enthalten. Hierfür kommen grundsätzlich beispielsweise Verfahren der Biosynthese mittels Mikroorganismen in Frage. Die heterologe Expression von Membranproteinen aus Säugerzellen in Wildtypstämmen ist jedoch aufgrund des

Fehlens von Cholesterol, bzw. der Anwesenheit von Ergosterol häufig mit Schwierigkeiten verbunden, da die exprimierten Membranproteine oftmals funktionell eingeschränkt bzw. inaktiv sind. Begründet werden kann dies durch die bekannte Tatsache, dass Sterole in den Membranen eine wichtige Rolle bei der korrekten Faltung und der Induktion einer aktiven Konformation von Membranproteinen und somit ihrer Funktionalität spielen, d.h. für eine funktionelle Expression von heterologen Membranproteinen in einer Hefe muss das Sterolmuster der Membranen zum Membranprotein passen.

Sterole sind ein wesentlicher Bestandteil der Membranen eukaryotischer Zellen. Sie sind verantwortlich für die Fluidität und Permeabilität der Membranen. Besonders hervorzuheben ist ihre Beteiligung an der Regulation zahlreicher Membranproteine. Hierbei sind sie als eine Art Kofaktoren für die korrekte Faltung, Stabilität und Aktivität der Membranproteine von Bedeutung. Freie Sterole sind in eukaryotischen Zellen in der Plasmamembran, sowie den Membranen aller Zellkompartimente zu finden. In den Lipidpartikeln liegen Sterole in veresterter Form als Speicherlipide vor. In Hefen ist der größte Teil der freien Sterole in der Plasmamembran lokalisiert, gefolgt von den sekretorischen Vesikeln, wobei die Menge in Mikrosomen, Vakuolen und Mitochondrienmembranen gering ist. Ergosterol stellt das Endprodukt des Sterol-Biosynthesewegs beispielsweise in der Hefe S. cerevisiae dar und ist das Hauptsterol in der Plasmamembran und den sekretorischen Vesikeln. Die Membranen der subzellulären Kompartimente enthalten geringere Mengen an Sterolen, aber auch andere Sterolintermediate.

In Säugerzellen ist das Endprodukt der Sterol-Biosynthese Cholesterol, welches den größten Teil der Sterole in der Plasmamembran ausmacht. Die Plasmamembran enthält etwa 60-80% des gesamten zellulären Cholesterols, das ER, der Ort der Sterolsynthese, nur etwa 0,5-1%.

Beispielsweise in der Literaturstelle Wildt, S., Gerngross, T. U. Nature Reviews (2005) 3: 119-128 ist von einer Humanisierung von Hefen die Rede. Der Begriff der Humanisierung bezieht sich jedoch dabei auf das Glykosylierungsmuster von heterolog produzierten Proteinen, welches dem des Menschen angepasst wurde und nicht auf das im Rahmen der vorliegenden Erfindung relevante Sterolmuster. Primär ist die Synthese von Enzymen oder Antikörpern und nicht von Membranproteinen behandelt.

Der Ergosterol-Biosyntheseweg von Hefen lässt sich in den Präsqualenweg, also die Synthese von Squalen aus Acetyl-CoA Molekülen, und den Postsqualenweg, in dem die Umsetzung von Squalen zu Ergosterol katalysiert wird, unterteilen. Als Hauptschrittmacherenzym des Präsqualenweges wurde die Hydroxymethylglutaryl-CoenzymA-Reduktase (HMG-CoA-Reduktase) identifiziert. Der sehr energieaufwendige Ergosterol-Biosyntheseweg wird vor allem durch dieses Enzym reguliert, welches dementsprechend zahlreichen Regulationsmechanismen, wie z.B. der Feedback-Hemmung durch Ergosterol, unterliegt. Der Präsqualen- und der Postsqualenweg, bis zur Synthese von Zymosterol, verlaufen in Hefe und Säugerzellen in der gleichen Weise. Die Unterschiede downstream von Zymosterol bis Ergosterol bzw. Cholesterol sind folgend erläutert. Bei Hefezellen wird Zymosterol durch C24-Methyltransferase zu Fecosterol, anschließend durch delta7-delta8-Isomerase zu Episterol, durch delta5-Desaturase zu Ergosta-5,7,24(28)-trienol, durch delta 22-Desaturase zu Ergosta-5,7,22,24(28)-tetraenol und schließlich durch delta 24-Reduktase zu Ergosterol umgesetzt. Bei Säugerzellen wird Zymosteröl durch delta7-delta8-Isomerase zu Cholesta-7,24-dienol, durch delta5-Desaturase zu Cholesta-5,7,24-trienol und durch delta7-Reduktase zu Desmosterol, und schließlich durch Sterol-delta5-desaturase zu Cholesterol umgesetzt. Dabei kann das letztgenannte Enzym auch Cholesta-7,24-dienol zu Lathosterol und Cholesta-5,7,24-trienol zu 7-Dehydro-cholesterol reagieren. Lathosterol wiederum kann durch delta5-Desaturase zu 7-Dehydrocholesterol reagiert werden. Letzteres wiederum reagiert durch delta7-Reduktase ebenfalls zu Cholesterol.

Ein Beispiel für ein humanes Membranprotein ist der Serotonin-Transporter (SERT). Er gehört zur Familie der Na⁺/Cl⁻ abhängigen Neurotransmitter-Transporter, welche für die Wiederaufnahme von biogenen Aminen aus dem synaptischen Spalt zurück in die praesynaptische Nervenendigung verantwortlich sind. Weitere Mitglieder dieser Familie sind die Transporter für Noradrenalin, Dopamin, Cholin, Glycin und γ-Aminobuttersäure. Der Serotonin-Transporter ist pharmakologisches Zielmolekül vieler Antidepressiva von hoher klinischer Bedeutung. Die Antidepressiva, welche den SERT als Ziel haben, lassen sich in zwei Hauptgruppen unterteilen: Zum einen die tricyclischen Wirkstoffmoleküle, wie z.B. Imipramin, Desimipramin, Clomipramin, und zum anderen die SSRI's (selective serotonin reuptake inhibitors). Zu letzterer Gruppe zählen Wirkstoffe wie z.B. Paroxetin, Fluoxetin, Sertralin und Citalopram.

Der SERT wurde bisher aus dem Gewebe des Menschen, der Ratte, der Maus, des Rinds und der Taufliege Drosophila melanogaster kloniert. Exprimiert wurde der SERT in allen gängigen Systemen, also in E. coli und der Hefe Pichia Pastoris, in Insektenzellen und in verschiedenen Zelllinien, wie zum Beispiel COS-1 Zellen, BHK-Zellen, Hela-Zellen und HEK-Zellen. Eine Expression des SERT in der Hefe Saccharomyces cerevisiae ist unbekannt. Eine funktionelle Expression ist bislang nur in Säuger-Zelllinien, sowie in Insektenzellen gelungen, nicht jedoch in E. coli oder Pichia pastoris. Der SERT ist ein äußerst schwieriges Protein im Bezug auf die heterologe bzw. Überexpression. Schwierigkeiten entstehen vor allem durch die Abhängigkeit des Serotonin-Transporters von Cholesterol in der das Protein umgebenden Membran und der Bedeutung der Glykosilierung für die richtige Faltung des Proteins. Eine Aktivität des Serotonin-Transporters ist nur nachweisbar, wenn Cholesterol in der Membran vorhanden ist. Vermutlich induziert dieses Sterol einen konformellen Zustand des SERT, welcher optimal für dessen Aktivität ist. Bislang wird vermutet, dass das Fehlen von Cholesterol in der Membran nicht durch Substitution mit anderen Sterolen kompensiert werden kann. Aufgrund der Bedeutung des Cholesterols für den SERT, erscheint klar, warum eine funktionelle Expression in E. coli bisher gescheitert ist. Über Western-Blot Experimente konnten Tate et al. (Tate, C. G., Haase, J., Baker, C., Boorsma, M., Magnani, F., Vallis, Y., und Williams, D. C. Biochimica et Biophysica Acta (2003) 1610, 141-153) zeigen, dass der rSERT in Pichia Pastoris zwar glykosiliert, aber nicht funktionell exprimiert wird. Die Vermutung liegt nahe, dass das Fehlen von Cholesterol in der Hefe dafür verantwortlich ist.

Die Literaturstellen Xu SH, Nes WD, Biochem Biophys Res Commun. (1988) 155 (1): 509-17 und WO-2005/121315 A beschreiben Cholesterol produzierende Hefen. Aus der Literaturstelle US-2005/00544108 A1 ist in anderen Zusammenhängen ein Hefe-basiertes System zur Verwendung in Screeningverfahren bekannt.

Ein modifizerter Organismus, beispielsweise ein Stamm der Hefe Saccharomyces cerevisiae, welcher die Fähigkeit besitzt Cholesterol oder eine Vorstufe zu synthetisieren, welche das Fehlen von Cholesterol bezüglich des Serotonin-Transporters kompensieren kann, würde eine funktionelle Expression dieses Proteins in nicht-Säugern erstmals möglich erscheinen lassen. Im Falle einer funktionellen Expression könnte ein solcher Organismus als Grundlage für die Entwicklung eines "Bioassays" genutzt werden. Mit diesem "Bioassay" könnten, durch das "Screening" verschiedener Stoffgruppen, neue, pharmakologisch relevante Wirkstoffe für den Serotonin-Transporter identifiziert werden. Des Weiteren könnte ein solcher Hefestamm auch für die funktionelle Expression anderer Cholesterol-abhängiger Membranproteine eingesetzt werden. Die Liste der humanen Membranpoteine, für welche eine Cholesterolabhängigkeit nachgewiesen wurde oder vermutet wird, ist lang und nimmt stetig zu. Die Liste der Krankheiten und Infektionen, in deren Entstehung oder Verlauf Cholesterol-abhängige Proteine involviert sind, ist ebenso lang. Ein als Plattform zur Expression dieser Proteine geeignetes System, könnte folglich von größtem Wert sein.

### Technisches Problem der Erfindung.

Der Erfindung liegt daher das technische Problem zu Grunde, einen nicht-Säuger Organismus, insbesondere einen Mikroorganismus, zu schaffen, in welchem Säuger-Membranproteine, insbesondere humane Membranproteine, effizient und einfach funktional exprimiert werden können.

Der Erfindung liegt das weitere technische Problem zu Grunde, ein Mittel zu schaffen, welche auf einfache und effiziente Weise das Screenen von Substanzen bzw. Substanzbibliotheken nach Agonisten oder Antagonisten der Säuger-Membranproteine insbesondere der humanen Membranproteine, erlauben.

Grundzüge der Erfindung und bevorzugte Ausführungsformen.

Zur Lösung dieses technischen Problems lehrt die Erfindung den Gegenstand des Anspruchs 1.

Die Erfindung beruht auf der überraschenden Erkenntnis, dass für eine funktionale Expression von Säuger-Membranproteinen, i.e. der Expression in einer Faltung, welche die enzymatische Aktivität des Membranproteins fördert bzw. erst ermöglicht, nicht nur die Anwesenheit von Cholesterol nützlich bzw. erforderlich ist, sondern dass vielmehr der gleiche Erfolg auch bereits bei Anwesenheit von Desmosterol erzielbar ist. Zudem zeigt es sich, dass das Desmosterol tatsächlich auch in der (Plasma) Membran des Organismus lokalisiert ist. Es ist also ein nicht-Säuger Organismus geschaffen, welcher in einer Membran, insbesondere der Plasmamembran, einen gegenüber dem Wildtyp erhöhten Gehalt an Desmosterol und ggf., wie folgend noch erläutert, an Cholesterol aufweist. Mit der Erfindung wird letztendlich erreicht, dass Säuger-Membranproteine, insbesondere humane Membranproteine, in nicht-Säuger-Organismen, beispielsweise in Hefen, funktional heterolog exprimiert werden können, indem diese Organismen zusätzlich mit einem Gen zur Expression des Membranproteins transformiert werden. Die Membranproteine können somit in hoher Menge und einfacher sowie kostengünstige Weise funktional hergestellt werden. Dies kann auf verschiedenste Weisen weiter genutzt werden. Neben der Synthese zu Herstellungszwecken oder für strukturelle und/oder funktionale Studien kann ein erfindungsgemäßer Organismus beispielsweise auch dazu verwendet werden, an das (funktionelle) Membranprotein bindende Substanzen, beispielsweise durch Screenen, zu identifizieren. Ein erfindungsgemäßer Organismus ist daher ein universelles Instrument zur funktionellen Synthese praktisch beliebiger Membranproteine, beispielsweise Plasmamembranproteine, aus Säugern, wie Menschen, sowie zur Findung von Agonisten und/oder Antagonisten solcher Membranproteine. Die Erfindung hat daher auch besondere Bedeutung als Instrument zur Findung pharmazeutisch brauchbarer Substanzen.

Mit der genetischen Veränderung des Organismus in Hinblick auf erhöhte HMG-CoA-Reduktase Aktivität wird eine erhöhte Produktion von Zymosterol erreicht. Mit der genetischen Veränderung in Hinblick auf reduzierte C24-Methyltransferase- und/oder delta22-Desaturase-Aktivität, vorzugsweise für beide reduziert, wird erreicht, dass die Biosynthese von Ergosterol blockiert bzw. reduziert wird, und dass stattdessen mit den ohnehin im Organismus vorhandenen Aktivitäten an delta7-delta8-Isomerase und delta5-Desaturase, zusammen mit der eingeführten delta7-Reduktase-Aktivität, die Reaktion von Zymosterol über Cholesta-7-24-dienol und Cholesta-5,7,24-trienol zu Desmosterol katalysiert wird.

In einer Weiterbildung der Erfindung kann vorgesehen sein, dass der Organismus zusätzlich eine gegenüber dem Wildtyp erhöhte Dehydrocholesterol-delta24-Reduktase-Aktivität aufweist. Dadurch erfolgt eine weitere Umsetzung sowohl des Desmosterols zu Cholesterol als auch der Desmosterol-Vorläufer Cholesta-7,24-dienol und Cholesta-5,7,24-trienol zu den Cholesterol Vorläufern Lathosterol und 7-Dehydro-cholesterol.

Bevorzugt ist es des Weiteren, wenn der Organismus eine gegenüber dem Wildtyp erhöhte Squalen-Epoxidase- und/oder Lanosterol-Demethylase-Aktivität aufweist. Dadurch wird die Biosynthese von Zymosterol aus Squalen gefördert und daher folglich auch die Produktion von Desmosterol bzw. Cholesterol.

Als nicht-Säuger-Organismus kommt grundsätzlich jeder beliebige Organismus in Frage. Der Organismus kann ein prokaryotischer Organismus, insbesondere ausgewählt aus der Gruppe bestehend aus Bakterien der Gattungen Bacillus, Escherichia, Lactobacillus, Corynebacterium, Acetobacter, Acinetobacter und Pseudomonas oder ein eukaryotischer Organismus, insbesondere ausgewählt aus der Gruppe bestehend aus Algen der Gattungen Cryptista, Chloromonadophyceae, Xanthophyceae, Crypthecodinium, Chrysophyta, Bacillariophyta, Phaeophyta, Rhodophyta, Chlorophyta, Haptophyta, Cryptista, Euglenozoa, Dinozoa, Chlorarachniophyta, Hefen, Insektenzellen aus Spodoptera frugiperda, Trichoplusia ni, Mamestra brassicae, Drosophila, sowie Pilze der Gattungen Aspergillus, Penicillium, Rhizopus, Fusarium, Fusidium, Gibberella, Mucor, Mortierella, Trichoderma oder Pflanzen wie Erdnuss, Raps, Canola, Sonnenblume, Safflor (Färberdistel), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen- Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte sein. Im Falle einer Hefe kann diese ausgewählt sein aus der Gruppe bestehend aus Saccharomyces cerevisiae, Saccharomyces delbrückii, Saccharomyces italicus, Saccharomyces ellipsoideus, Saccharomyces fermentati, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum und Saccharomycodes ludwigii, sowie Hefen der Gattung Kluyveromyces wie K. lactis K. marxianus var. marxianus, K. thermotolerans, sowie Hefen der Gattung Candida wie Candida utilis, Candida tropicalis, Candida albicans, Candida lipolytica und Candida versatilis, sowie Hefen der Gattung Pichia wie Pichia stipidis, Piachia pastoris und Pichia sorbitophila, sowie Hefen der Gattungen Cryptococcus, Debaromyces, Hansenula, Saccharomycecopsis, Saccharomycodes, Schizosaccharomyces, Wickerhamia, Debayomyces, Hanseniaspora, Kloeckera, Zygosaccharomyces, Ogataea, Kuraishia, Komagataella, Metschnikowia, Williopsis, Nakazawaea, Cryptococcus, Torulaspora, Bullera, Rhodotorula, Willopsis und Sporobolomyces und Moose wie Physcomitrella oder Ceratodon.

Der vorstehend beschrieben Organismus bildet die Basis bzw. ein Werkzeug zur Schaffung eines zusätzlich genetisch modifizierten Organismus, welcher ein Säuger-Membranprotein funktional heterolog exprimiert. Daher ist es weiterhin bevorzugt, wenn der Organismus zusätzlich transformiert ist mit einem für ein Membranprotein eines Säugers, insbesondere ein humanes Membranprotein, vorzugsweise ein Plasmamembranprotein, codierenden Gen, unter Kontrolle eines vorzugsweise konstitutiv aktiven Promotors. Das für das Membranprotein codierende Gen ist dabei grundsätzlich beliebig und ist vorzugsweise ausgewählt aus der Gruppe oder Familie bestehend aus Serotonin-Transporter-Gen, Noradrenalin-Transporter-Gen, Dopamin-Transporter-Gen, Cholin-Transporter-Gen, Glycin-Transporter-Gen, gamma-Aminosäure-Transporter-Gen, 5-hydroxytryptamine receptor 3 subunit C , Mast/stem cell growth factor receptor precursor , Toll-like receptor 8 precursor , similar to olfactory receptor MOR233-18 , greensensitive opsin , glycine receptor, frizzled 5 precursor, dimethylaniline monooxygenase [N-oxide forming], calcitonin gene-related peptide type 1 receptor precursor, muscarinic acetylcholine receptor, C-C chemokine receptor type 4, G protein-coupled receptor, receptor tyrosine kinase , dopamine receptor , substance-K receptor , putative neurotransmitter receptor , thyroid hormone receptor-associated protein complex component TRAP230, fMet-Leu-Phe receptor, gammaaminobutyric-acid receptor alpha-6 subunit precursor (GABA(A) receptor), glucagon-like peptide 2 receptor precursor, G protein-coupled receptor 56, EMR1 hormonereceptor, CCK-B/gastrin receptor, alpha-2C-adrenergic receptor, D1beta dopamine receptor, probable G protein-coupled receptor GPR72 precursor , relaxin receptor 2, similar to phospholipase A2 receptor 1, 180kD, coagulation factor II receptor precursor, somatostatin receptor type 1, acetylcholine receptor protein, alpha chain precursor, interleukin-7 receptor alpha chain precursor, similar to interleukin 9 receptor, similar to transmembrane receptor Unc5H1, ER lumen protein retaining receptor 2, FGF receptor activating protein 1, bone morphogenetic protein receptor type IB precursor, prolactin receptor precursor, tumor necrosis factor receptor, similar to Homo sapiens multiple membrane spanning receptor TRC8 mRNA, feline leukemia virus subgroup C receptor FLVCR, interferon-alpha/beta receptor beta chain precursor, polymeric-immunoglobulin receptor precursor, cell surface glycoprotein receptor CD200, similar to low density lipoprotein receptor-related protein 3, atrial natriuretic peptide receptor B precursor, signal sequence receptor-alpha, protein tyrosine phosphatase, non-receptor type 21, I-1 receptor candidate protein, probable G protein-coupled receptor GPR37 precursor, interleukin-10 receptor alpha chain precursor , very large G protein-coupled receptor 1, G-protein-coupled receptor HE6 precursor, similar to glutamate receptor delta-1 subunit, retinoic acid receptor gamma-1, similar to olfactory receptor MOR42-1, similar to calciumsensing receptor related protein 3, autocrine motility factor receptor precursor, class I cytokine receptor, similar to nogo receptor, 5-hydroxytryptamine 6 receptor, tumor necrosis factor receptor super member 1B precursor, polycystic kidney disease and receptor for egg jelly related protein, putative G-protein coupled receptor, sortilin-related receptor precursor, interleukin-12 receptor beta-1 chain precursor, c-type lectin-like receptor-2, similar to olfactory receptor, tumor necrosis factor receptor super member TNFRSF19L precursor, similar to cadherin EGF LAG seven-pass G-type receptor 2, G-protein-coupled receptor, protein tyrosine phosphatase, receptor type, W, folate receptor alpha precursor, G protein-coupled receptor, similar to olfactory receptor MOR217-1, guanine nucleotide-binding protein-like 1, tumor necrosis factor receptor super member 21 precursor, neuronal acetylcholine receptor protein, alpha-7 chain precursor, interleukin-2 receptor beta chain precursor, cation-dependent mannose-6-phosphate receptor precursor, interleukin-17 receptor precursor, similar to Gammaaminobutyric acid type B receptor, subunit 2 precursor, interleukin-2 receptor alpha chain precursor, tumor necrosis factor receptor super member 18 precursor, similar to G protein-coupled receptor, C, group 5, similar to MrgG G protein-coupled receptor, interleukin-6 receptor beta chain precursor, lymphatic endothelium-specific hyaluronan receptor LYVE-1, putative G-protein coupled receptor, TGF-beta receptor type III precursor, similar to golgi SNAP receptor complex member 1, high affinity immunoglobulin epsilon receptor alpha-subunit precursor, interleukin-4 receptor alpha chain precursor, interleukin-12 receptor beta-2 chain precursor, similar to leucocyte immunoglobulin-like receptor-1, similar to P2Y purinoceptor 3 (P2Y3) (Nucleoside diphosphate receptor), tumor necrosis factor receptor super member 11A precursor, lymphatic endothelium-specific hyaluronan receptor LYVE-1, activating receptor PILRbeta, ephrin type-A receptor 7 precursor, similar to probable thromboxane A2 receptor isoform beta -human, protein tyrosine phosphatase, receptor type, C, isoform 2 precursor, endothelial protein C receptor precursor, ATP-binding cassette, sub- B, Putative ATP-binding cassette transporter C13, ATP-binding cassette transporter , ATP-binding cassette, sub- A (ABC1), ATP-binding cassette, sub B , similar to multidrug resistance protein 2, ATP-binding cassette, sub- D , ATP-binding cassette, sub- A, ATP-binding cassette, sub- G, Similar to solute carrier 21 (organic anion transporter), organic anion transporter 3, solute carrier 2, (facilitated glucose transporter), solute carrier 7, (cationic amino acid transporter, y+ system), HCAT-2A, solute carrier 6 (neurotransmitter transporter, glycine), sodium-dependent high-affinity dicarboxylate transporter, similar to solute carrier 26, solute carrier 21, UDP N-acetylglucosamine transporter, neutral amino acid transporter B(0), solute carrier 2, facilitated glucose transporter, solute carrier 29 (nucleoside transporters), member 1, solute carrier 26, solute carrier 10 (sodium/bile acid cotransporter ), H+/peptide transporter, similar to solute carrier 9, solute carrier 30, solute carrier 25, solute carrier 19 (folate transporter), UDP-glucuronic acid/UDP-N-acetylgalactosamine transporter, solute carrier 16 (monocarboxylic acid transporters), member 4, nucleoside transporter, sodium/nucleoside cotransporter 1, similar to Mus musculus putative thymic stromal co-transporter TSCOT mRNA, chromaffin granule amine transporter , acetyl-coenzyme A transporter, high affinity choline transporter, organic-cation transporter like, membrane-associated transporter protein, similar to amino acid transporter subunit B0,+AT, similar to non-green plastid triose phosphate translocator precursor, similar to peptide/histidine transporter, Rh type B glycoprotein, sodium/hydrogen exchanger 6, solute carrier 39 (zinc transporter), member 4, peroxisomal Ca-dependent solute carrier, K-CL cotransporter, solute carrier 4, anion exchanger, sodium/iodide cotransporter, chloride anion exchanger, similar to sodium/potassium/calcium exchanger 3 precursor (Na(+)/K(+)/Ca(2+)-exchange protein 3), sodium dependent phosphate transporter, similar to solute carrier 20 (phosphate transporter, solute carrier 8 (sodium-calcium exchanger), solute carrier 11 (sodium/phosphate symporters), weakly similar to metal homeostasis factor ATX2, ironregulated transporter IREG1, probable low-affinity copper uptake protein 2, similar to solute carrier 9 (sodium/hydrogen exchanger), isoform 7, Voltage-gated sodium channel alpha subunit, potassium channel TASK-4, small conductance calcium-activated potassium channel protein 2, long transient receptor potential channel 2, ether-a-go-go-like potassium channel 1, voltage-gated potassium channel protein, sodium channel, nonvoltage-gated 1, delta, voltage-dependent N-type calcium channel, aquaporin, calcium channel, voltage-dependent, alpha, similar to transient receptor potential cation channel , ryanodine receptor 3, transient receptor potential cation channel, potassium channel sub K, cyclic nucleotide gated channel alpha, similar to Rattus norvegicus potassium channel regulator 1 mRNA, similar to chloride channel protein 3 (C1C-3), calcium-activated chloride channel-2, lens fiber membrane intrinsic protein, sperm ion channel, similar to Shaw-related potassium channel protein Raw1, dihydropyridine-sensitive L-type, calcium channel alpha-2/delta subunits precursor, similar to voltage-dependent anion channel 2, mid-1-related chloride channel, similar to potassium channel subunit (Slack), ATPase, Ca++ transporting, fast twitch 1, plasma membrane calciumtransporting, vacuolar proton translocating ATPase 116 kDa subunit A, sodium/potassium-transporting ATPase alpha-3 chain, copper-transporting ATPase 1 (Copper pump 1) (Menkes disease-associated protein), probable cation-transporting ATPase 3 (Fragment), vacuolar ATP synthase subunit S1 precursor, similar to Potential phospholipid-transporting ATPase ID, sodium/potassium-transporting ATPase beta-3 chain, tyrosine kinase, protein-tyrosine phosphatase, non-receptor type 5, putative transmembrane GTPase (Fragment), epidermal growth factor (beta-urogastrone), protein kinase C-like 1, weakly similar to RAS suppressor protein 1, dioxin receptor repressor, cAMP-spec,ific 3',5'-cyclic phosphodiesterase 4A, MAP-kinase activating death domain-containing protein, isoform g, ras guanine nucleotide exchange factor 2 (Fragment), NADPH-dependent FMN and FAD containing oxidoreductase, dolichyl-diphosphooligosaccharide--protein glycosyltransferase 63 kDa subunit precursor, phosphatidylinositol glycan, class B, A kinase (PRKA) anchor protein 13, 85 kDa calcium-independent phospholipase A2, A-Raf proto-oncogene serine/threonine-protein kinase, similar to peptidylprolyl isomerase A (cyclophilin A), Ras-related GTP-binding protein, similar to sphingosine-1-phosphate phosphatase 1, WNT-10A protein precursor, similar to ADPribosylation factor-like 1, transforming growth factor alpha precursor, guanine nucleotide exchange factor, protein-tyrosine phosphatase, non-receptor, similar to putative serine/threonine protein kinase, calcium/calmodulin-dependent protein kinase type IV catalytic chain, similar to phosphatidylinositol-4-phosphate 5-kinase, type II, gamma, similar to 52 kDa repressor of the inhibitor of the protein kinase (p58IPK-interacting protein) (58 kDa interferon-induced protein kinase-interacting protein) (P52rIPK) (Death associated protein 4), apoptosis regulator, SH2 domain-containing phosphatase anchor protein 1, similar to RHO-GAP hematopoietic protein C1, similar to phosphatidylinositol 3-kinase delta catalytic subunit, similar to tumor-associated calcium signal transducer 1 precursor (Major gastrointestinal tumor-associated protein GA733-2) (Epithelial cell surface antigen) (Epithelial glycoprotein) (EGP) (Adenocarcinomaassociated antigen) (KSA) (KS 1/4 antigen), proto-oncogene tyrosine-protein kinase ROS precursor, membrane-bound transcription factor site 2 protease, inositol polyphosphate 5-phosphatase, microsomal signal peptidase 25 kDa subunit, similar to AMP-activated protein kinase gamma subunit, tumor necrosis factor ligand super member 8, sterol regulatory element binding protein cleavage-activating protein, transmembrane protease, serine 3, isoform 3, similar to Ser-Thr protein kinase related to the myotonic dystrophy protein kinase, membrane-bound transcription factor site-1 protease precursor, similar to 52 kDa repressor of the inhibitor of the protein kinase (p58IPK-interacting protein) (58 kDa interferon-induced protein kinase-interacting protein) (P52rIPK) (Death associated protein 4), PAS-kinase, serine/threonine protein phosphatase 2B catalytic subunit, alpha isoform, signal transduction protein CBL, rab proteins geranylgeranyltransferase component A 2, similar to Rhointeracting protein 2 (Rho-guanine nucleotide exchange factor) (RhoGEF) (RIP2), pre-mRNA splicing factor ATP-dependent RNA helicase PRP16, splicing factor 3b, subunit 1, 155kD, SP110 nuclear body protein, isoform a, similar to SMC2 structural maintenance of chromosomes 2-like 1 (yeast), similar to polymerase (DNA directed), theta, nuclear factor I/B, nurim (nuclear envelope membrane protein), similar to nuclear receptor coactivator 4, weakly similar to splicing factor, arginine/serine-rich 4, similar to Elongation factor 1-alpha 1 (EF-1-alpha-1) (Elongation factor 1 A-1) (eEF1A-1) (Elongation factor Tu) (EF-Tu), similar to 60S ribosomal protein L7, deoxyribonuclease II alpha putative, nuclear factor of kappa light polypeptide gene enhancer in B-cells inhibitor, epsilon, solute carrier 25 (mitochondrial carrier), similar to cytochrome b-561, cytochrome oxidase subunit I , succinyl-CoA ligase [GDP-forming] beta-chain, mitochondrial precursor (Fragment), similar to Cytochrome oxidase biogenesis protein OXA1, mitochondrial precursor (OXA1-like protein) (OXA1Hs), NADH-Ubiquinone Oxidoreductase Chain 5, trifunctional enzyme beta subunit, mitochondrial precursor, methylcrotonyl-CoA carboxylase beta chain, mitochondrial precursor, similar to cytochrome c oxidase subunit III, putative ATP-dependent Clp protease proteolytic subunit, mitochondrial precursor, NADH-ubiquinone oxidoreductase 39 kDa subunit, mitochondrial precursor, second mitochondria-derived activator of caspase, isoform Smac-delta, precursor, similar to NADH dehydrogenase subunit 5, mitochondrial import inner membrane translocase subunit TIM23, similar to cytochrome b, mitochondrial precursor proteins import receptor, Cytochrome c oxidase polypeptide II, short chain 3-hydroxyacyl-CoA dehydrogenase, mitochondrial precursor, similar to 60 kDa heat shock protein, mitochondrial precursor (Hsp60), Cytochrome c1, heme protein, mitochondrial precursor, protoheme IX farnesyltransferase, mitochondrial precursor, glycerol-3-phosphate dehydrogenase, mitochondrial precursor, homeobox protein Hox-A7, transcriptional regulator ATRX, similar to general transcription factor II, i, isoform 1, similar to general transcription factor IIIC, polypeptide 1 (alpha subunit, 220kD ), transcriptional activator SRCAP, sterol/retinol dehydrogenase, alkaline phytoceramidase, carnitine O-palmitoyltransferase I, mitochondrial liver isoform , similar to bA84N7.1 (novel protein similar to diacylglycerol kinase eta (DGKH)), 1-acyl-sn-glycerol-3-phosphate acyltransferase epsilon, mannose-P-dolichol utilization defect 1 protein, sterol O-acyltransferase 2, apolipoprotein L1 precursor, phosphatidylcholine-sterol acyltransferase precursor, fatty acid hydroxylase, moderately similar to Mus musculus putative lysophosphatidic acid acyltransferase mRNA, weakly similar to phospholipase A2 inhibitor subunit B precursors, phosphatidylinositol-glycan biosynthesis, class F protein , serine palmitoyltransferase 1, phosphatidylserine synthase I, similar to 1-acyl-sn-glycerol-3-phosphate acyltransferase gamma (1-AGP acyltransferase 3) (1-AGPAT 3) (Lysophosphatidic acid acyltransferase-gamma) (LPAAT-gamma) (1-acylglycerol-3-phosphate O-acyltransferase 3), similar to Triacylglycerol lipase, gastric precursor (Gastric lipase) (GL), 3-hydroxy-3-methylglutaryl-coenzyme A reductase, ceramide glucosyltransferase, dolichyl-P-Man:Man(5)GlcNAc(2)-PP-dolichyl mannosyltransferase, N-acetylglucosaminylphosphatidylinositol de-N-acetylase, dolichyl-diphosphooligosaccharide--protein glycosyltransferase 67 kDa subunit precursor, diacylglycerol kinase, zeta, apolipoprotein F, similar to fatty acid amide hydrolase, similar to Long-chain-fatty-acid--CoA ligase 6 (LACS 6), farnesyl pyrophosphate synthetase (FPP synthetase), sphingomyelin phosphodiesterase 2, neutral membrane (neutral sphingomyelinase), estradiol 17 beta-dehydrogenase 7, phosphatidyl inositol glycan class S, 24-dehydrocholesterol reductase precursor, oxysterol-binding protein 1, 1-acyl-sn-glycerol-3-phosphate acyltransferase beta, myo-inositol-1(or 4)-monophosphatase, estradiol 17 beta-dehydrogenase7, 1-acyl-sn-glycerol-3-phosphate acyltransferase beta, putative fatty acid desaturase MLD, CDP-diacylglycerol--inositol 3-phosphatidyltransferase, lysosomal apyrase-like protein 1, acyl-malonyl condensing enzyme, ubiquitin-conjugating enzyme E2, thyroid peroxidase precursor (2 members), UDP-N-acetylglucosamine--dolichyl-phosphate N-acetylglucosaminephosphotransferase, N-acetylglucosamine-1-phosphodiester alpha-N-acetylglucosaminidase , 3 betahydroxysteroid dehydrogenase/delta 5-->4-isomerase type II, similar to adenylate cyclase 6, isoform a, similar to cytochrome P450 IID6, GalNAc 4-sulfotransferase, GPI transamidase, proprotein convertase subtilisin/kexin type 7, NEDD4-like ubiquitin ligase 1, hyaluronan synthase 2, ketohexokinas, testis ecto-ADP-ribosyltransferase precursor, alkaline phosphatase, intestinal precursor, 5,6-dihydroxyindole-2-carboxylic acid oxidase precursor, biotinprotein ligase, dolichyl-diphosphooligosaccharide-protein glycosyltransferase, squalene monooxygenase, ubiquitin specific protease, similar to glycerol kinase, probable dolichyl pyrophosphate Glc1Man9GlcNAc2 alpha-1,3-glucosyltransferase, gamma-glutamyltranspeptidase 1 precursor, serine hydrolase-like protein, phosphatidate cytidylyltransferase 2, DNA topoisomerase II, beta isozyme, lysosomal acid phosphatase precursor, muscle-specific DNase I-like precursor, similar to kynurenine 3-monooxygenase, similar to Peptidylglycine alpha-amidating monooxygenase, N-acetylglucosamyl transferase component GPI1, similar to cGMP-inhibited 3,5-cyclic phosphodiesterase A (Cyclic GMP inhibited phosphodiesterase A) (CGI-PDE A), beta-1,4 mannosyltransferase, cholinephosphotransferase 1, moderately similar to Homo sapiens neuropathy target esterase, CAAX prenyl protease 1 homolog, pantothenate kinase 1alpha, similar to N-acetylglucosaminyltransferase VI, Cytochrome P450 39A1, similar to Glyceraldehyde 3-phosphate dehydrogenase, liver, similar to Beta-1,4 N-acetylgalactosaminyltransferase, CAAX prenyl protease 2, beta-1,3-galactosyltransferase-6, hexosaminidase A, carbonic anhydrase-related protein 2 precursor, vitamin K-dependent gamma-carboxylase, malate dehydrogenase, cytoplasmic, mannosyltransferase , long form of N-acetylglucosamine-6-O-sulfotransferase, sialic acid-specific acetylesterase II, similar to uracil DNA glycosylase, beta-hexosaminidase beta chain precursor, similar to L-lactate dehydrogenase B chain (LDH-B), probable serine protease HTRA3 precursor, weakly similar to UDP-N-acetylglucosamine--peptide N-acetylglucosaminyltransferase 110 kDa subunit, neutral sphingomyelinase II, similar to putative N-acetyltransferase Camello 2 , weakly similar to NADPH-cytochrome P450 reductase, similar to ectonucleotide pyrophosphatase/phosphodiesterase 5, similar to 3-OXO-5-alpha-steroid 4-dehydrogenase 2, blood plasma glutamate carboxypeptidase precursor, xylosyltransferase II, casein kinase I, epsilon isoform, selenide,water dikinase 2, paraoxonase 2, similar to NADH-ubiquinone oxidoreductase MLRQ subunit, similar to ADAMTS-5 precursor (A disintegrin and metalloproteinase with thrombospondin motifs 5) (ADAM-TS 5) (ADAM-TS5) (Aggrecanase-2) (ADMP-2) (ADAM-TS 11), galactose-3-O-sulfotransferase, valyl-tRNA synthetase 2, ribonuclease H1, acetyl-CoA carboxylase 2, similar to alpha-L-iduronidase precursor, similar to ADAMTS-5 precursor (A disintegrin and metalloproteinase with thrombospondin motifs 5) (ADAM-TS 5) (ADAM-TS5) (Aggrecanase-2) (ADMP-2) (ADAM-TS 11), kunitz-type protease inhibitor 1 precursor, putative sialoglycoprotease type 2, ubiquitin-activating enzyme E1 homolog, prenylcysteine lyase precursor, dimeric dihydrodiol dehydrogenase, glycosyltransferase, integrin alpha-V precursor , axonemal dynein heavy chain (Fragment), copcoated vesicle membrane protein p24 precursor, protocadherin ARCADLIN, Keratin, type I cytoskeletal, nuclear envelope pore membrane protein, gamma-tubulin complex component 6, Cleft lip and palate associated transmembrane protein 1 , Sec61 homolog, presenilins associated rhomboid-like protein , putative prostate cancer tumor suppressor, signal recognition particle receptor ('docking protein'), HLA class I histocompatibility antigen, B-8 B*0801 alpha chain precursor, weakly similar to peregrine, HLA class II histocompatibility antigen, beta chain precursor, Kit ligand precursor, Ret finger protein 2, ASPP1 protein, neuronal membrane glycoprotein M6-a, membrane cofactor protein (CD46, trophoblast-lymphocyte cross-reactive antigen), vesicle trafficking protein, epithelial membrane protein-1, neurexin 3-beta precursor, intercellular adhesion molecule-3 precursor, presenilin 2, BCL2/adenovirus E1B 19-kDa proteininteracting protein 2, corticosteroid-binding globulin precursor, T-cell surface glycoprotein CD8 alpha chain precursor, capillary morphogenesis protein-2 precursor, trans-golgi network protein 2, bax inhibitor-1, vang-like 1 (van gogh, Drosophila), tuberous sclerosis 2 isoform 2, P protein, down syndrome cell adhesion molecule precursor, vascular cell adhesion protein 1 precursor, clathrin assembly lymphoid myeloid leukemia protein, kelch-like protein 2, myeloid/lymphoid or mixed-lineage leukemia (trithorax homolog, Drosophila), transmembrane 4 super, microtubuleassociated protein 1A, tetraspanin, synaptotagmin XI, spinster-Like Protein, integrin beta 1 isoform 1A precursor, weakly similar to patched protein homolog 1, pro-neuregulin-2 precursor, tumor metastasis-suppressor, sel-1 homolog precursor, 150 kDa oxygen-regulated protein precursor, muprotocadherin related protein splice variant, weakly similar to protein PTM1 precursor, weakly similar to cochlin precursor, similar to TRAM protein (Translocating chainassociating membrane protein), COP9 complex subunit 7A, calcium binding atopy-related autoantigen 1, similar to metaxin 1, envelope protein precursor, protein associated with Myc , ocular albinism type 1 protein , PL6 protein, unknown function but deleted in small cell lung cancer (Fragment), tumor endothelial marker 5 precursor, tumor suppressor protein DCC precursor, mucin , tumor endothelial marker 7-related precursor, zinc finger protein 179, alpha integrin binding protein 63, similar to Stromal interaction molecule 1 precursor , similar to Homo sapiens squamous cell carcinoma antigen recognized by T cell, P400 SWI2/SNF2-related protein, NgCAM-related related cell adhesion molecule (Fragment), similar to transmembrane protein induced by tumor necrosis factor alpha, similar to Apobec-1 complementation factor, APOBEC-1 stimulating protein, malignant cell expression-enhanced gene/tumor progression-enhanced gene, putative ferritin (Fragment), weakly similar to human DHHC-domain-containing cysteine-rich protein mRNA, similar to multiple endocrine neoplasia I, similar to HIRA protein (TUP1 like enhancer of split protein 1), envelope protein, transmembrane 7 super protein member 3 precursor, bladder cancer overexpressed protein, transmembrane protein vezatin, golgi apparatus protein 1 precursor, vascular noninflammatory molecule 2 precursor , E-selectin precursor, myeloid cell surface antigen CD33 precursor, similar to FH1/FH2 domains-containing protein (Formin homolog overexpressed in spleen) (FHOS), patched protein homolog 1, pannexin 3, scleroderma-associated autoantigen, ecotropic viral integration site 2B, alpha-sarcoglycan precursor, hematopoietic progenitor cell antigen CD34 precursor, hepatocellular carcinoma-associated antigen 112, neuropilin 1, basigin precursor, pregnancy-associated plasma preproprotein-A2, basement membrane-specific heparan sulfate proteoglycan core protein precursor, high-risk human papilloma viruses E6 oncoproteins targeted protein E6TP1 beta , highly similar to Rattus norvegicus db83 mRNA, transducinlike enhancer protein 2, protocadherin fat 2, Unc-93 related protein, retinoblastoma-associated factor 600, CD44 antigen (homing function and Indian blood group system), meprin A alpha-subunit precursor, similar to Podocalyxin-like protein 1 precursor, similar to Mus musculus ganglioside-induced differentiation associated protein 1, leucine-rich repeat protein LRRC3 precursor, endothelial and smooth muscle cellderived neuropilin-like protein, source of immunodominant MHC-associated peptides, fibulin 2 precursor, CD3Z antigen, zeta polypeptide, T-cell surface glycoprotein CD3 gamma chain precursor, pancreatic secretory granule membrane major glycoprotein GP2 precursor, signaling lymphocytic activation molecule precursor, alzheimer's disease amyloid A4 protein precursor, protein pM5 precursor, similar to dynein light chain-A, lymphocyte-activation protein 3 precursor, progressive ankylosis protein homolog, erythrocyte band 7 integral membrane protein, SYG1 protein, similar to SYNAPSINS IA and IB, similar to amyotrophic lateral sclerosis 2 (juvenile) chromosome region, candidate 2, polycystic kidney disease 1 protein, niemann-Pick C1 protein precursor, DNAJ protein homolog 1, thrombomodulin precursor, weakly similar to occluding, Occludin, similar to rennin, junctophilin type3, similar to homeotic protein CHox3 - chicken , similar to Glycolipid transfer protein (GLTP), proteasome subunit alpha type , extracellular matrix protein, delta-sarcoglycan, prominin-like protein 1 precursor, translocon-associated protein, delta subunit precursor, peroxisome biogenesis factor 1, neuroglycan C, SH3 adapter protein SPIN90, Mcd4p homolog, T lymphocyte activation antigen CD80 precursor, similar to calcium-binding protein CaBP7, tight junction protein ZO-1, dysferlin, dachshund 2, barttin, T-cell surface glycoprotein CD5 precursor, brain specific membrane-anchored protein precursor, similar to Fatty acid-binding protein, heart (H-FABP) (Muscle fatty acid-binding protein) (M-FABP) (Mammary-derived growth inhibitor) (MDGI), peroxisome assembly factor-1, pentaxin-related protein PTX3 precursor, neuropilin and tolloid like-1, cat eye syndrome critical region protein 6, peptidoglycan recognition protein-I-alpha precursor, signal recognition particle receptor beta subunit, similar to ralA binding protein 1, T-cell surface protein tactile precursor, transmembrane gamma-carboxyglutamic acid protein 4 precursor, JAW1-related protein MRVI1A long isoform, peroxisomal membrane protein PEX13, zona pellucida sperm-binding protein 2 precursor , similar to proteasome (prosome, macropain) subunit, beta type, 3, cell surface glycoprotein MUC18 precursor, similar to golgi autoantigen, golgin sub a, 2, ISS~homolog to INHIBITOR OF CARBONIC ANHYDRASE PRECURSOR~putative, similar to Alpha-2 catenin (Alpha-catenin related protein) (Alpha N-catenin), sterol regulatory element binding protein-1, similar to Transcription elongation factor S-II (Transcription elongation factor A), protocadherin 15 precursor, homolog of DROSOPHILA HEADCASE, calmin, oxysterol binding proteinrelated protein 8, Nesprin-1 alpha, SOX-13 protein, DNAdirected RNA polymerase I largest subunit, ANTHRACYCLINEassociated resistance ARX, similar to Matrin 3, blood vessel epicardial substance, wolframin, fibroblast growth factor-4 precursor, noggin precursor, transcobalamin I precursor, von Willebrand factor precursor, laminin beta-3 chain precursor, similar to CAP-binding protein complex interacting protein 2, calsyntenin-2, brother of CDO, hepatocellular carcinoma-associated antigen 137, testis-specific protein TEX28, megakaryocyte-enhanced gene transcript 1 protein, similar to vitelliform macular dystrophy (Best disease, bestrophin), similar to integrin alpha-2b chain precursor - human, mucin 2 precursor, male-specific lethal-3 homolog 1, protocadherin Flamingo 2, STRA6 isoform 1, cystinosin, ELK4 protein, isoform b, similar to apoptosis regulator bcl-x isoform - human, endoglin precursor, similar to 1 beta dynein heavy chain, similar to WNT-5A protein precursor, similar to H3.3 like histone MH321 - mouse, MHC class II regulatory factor RFX1, sarcospan, mandaselin long form, matrilin-2 precursor, similar to DESC1 protein, interphotoreceptor matrix proteoglycan 200, inhibin alpha chain precursor, interphotoreceptor matrix proteoglycan 200, male-specific lethal-3 homolog 1, nephrin precursor, matrilin-2 precursor, M130 antigen cytoplasmic variant 2 precursor, cleavage and polyadenylation specificity factor, 160 kDa subunit, blood vessel epicardial substance, transmembrane 6 super member 2 (Fragment), similar to Zinc finger protein HRX (ALL-1) (Trithorax-like protein), NgCAM-related related cell adhesion molecule (Fragment), polycystin-1L1, similar to Dynamin-1, similar to OTK27, platelet glycoprotein Ib beta chain precursor, NMD protein, intersectin 2, matrilin-2 precursor, ERGIC-53 protein precursor, translocon-associated protein, gamma subunit, weakly similar to zinc/cadmium resistance protein, quiescin, similar to prot GOR, megakaryocyte-enhanced gene transcript 1 protein.

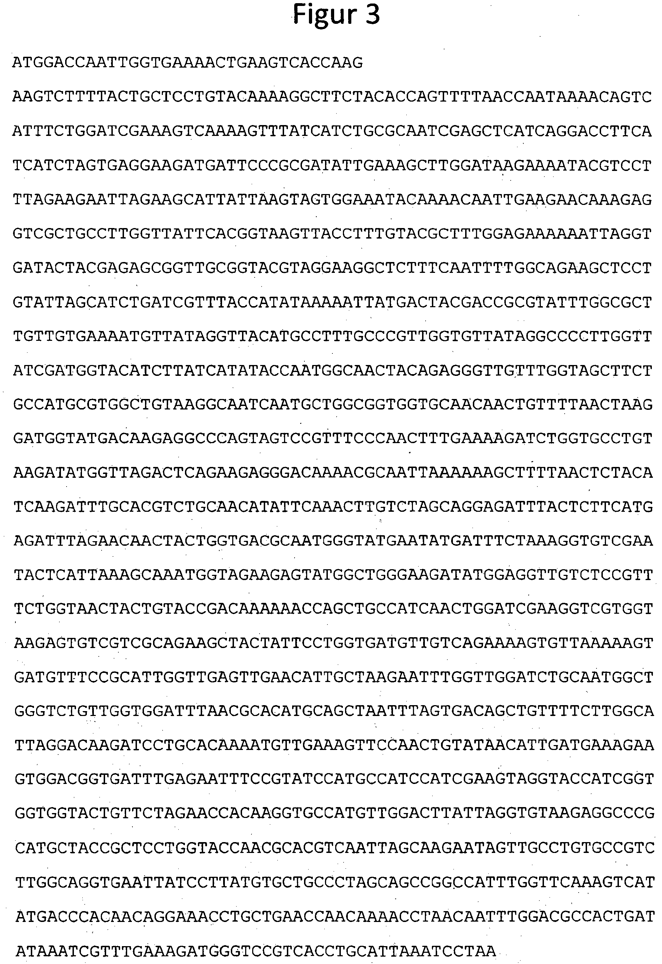

Gensequenzen codierend für geeignete HMG-CoA-Reduktasen, einschließlich trunkierter, aber noch funktionaler HMG-CoA-Reduktasen wie tHMG1, sind bekannt beispielsweise aus den folgenden Acc.-No. der Gendatenbank The National Center for Biotechnology Information (NCBI): NC_001145, NM_106299, NC_003421.2, NC_009784.1, NC_003028.3, NC_007308.3, sowie der Figur 3 (tHMG1).

Gensequenzen codierend für zu hemmende C24-Methyltransferasen sind bekannt beispielsweise aus den folgenden Acc.-No. der Gendatenbank The National Center for Biotechnology Information (NCBI): NC_001145, NC_000911.1, NC_003423.3.

Gensequenzen codierend für zu hemmende delta22-Desaturasen sind bekannt beispielsweise aus den folgenden Acc.-No. der Gendatenbank The National Center for Biotechnology Information (NCBI): NC_003424.3, NC_009046.1, NC_001145.2

Gensequenzen codierend für geeignete delta7-Reduktasen sind bekannt beispielsweise aus den folgenden Acc.-No. der Gendatenbank The National Center for Biotechnology Information (NCBI): NM_103926, NM_001360, NM_0078S6, NM_203904, NM_001014927, NM_201330, NM_022389, NM_001131727, NM_001087087, XM_001497S98, XM_001174160, XM_001099101, BM490402, CA753545

Gensequenzen codierend für geeignete delta24-Reduktasen sind bekannt beispielsweise aus den folgenden Acc.-No. der Gendatenbank The National Center for Biotechnology Information (NCBI): NM_014762, NM_001016800, NM_001094456, NM_001008645, NM_001103276, NM_001080148, NM_053272, NM_00103128, XM_001488247, AB125202, XM_001153751.

Gensequenzen codierend für geeignete delta7-delta8-Isomerasen sind bekannt beispielsweise aus den folgenden Acc.-No. der Gendatenbank The National Center for Biotechnology Information (NCBI): M74037,

Gensequenzen codierend für geeignete delta5-Desaturasen sind bekannt beispielsweise aus den folgenden Acc.-No. der Gendatenbank The National Center for Biotechnology Information (NCBI): S46162, NG_009446, NM_053642, NM_001035356.

Gensequenzen codierend für geeignete Squalen-Epoxidasen sind bekannt beispielsweise aus den folgenden Acc.-No. der Gendatenbank The National Center for Biotechnology Information (NCBI): M64994

Gensequenzen codierend für geeignete Lanosterol-Demethylasen sind bekannt beispielsweise aus den folgenden Acc.-No. der Gendatenbank The National Center for Biotechnology Information (NCBI) : EF059165 Gensequenzen codierend für beispielhaft heterolog exprimierbare Säuger-Membranproteine sind bekannt beispielsweise aus den folgenden Acc.-No. der Gendatenbank The National Center for Biotechnology Information (NCBI): NP_570126, NP_006019, NP_000860, NP_619542, NP_000786, NP_000787, NP_150645, NP_150646, NP_057658, NP_387512, NP_000669, NP_150647, NP_000671, NP_387507, NP_387508, NP_387509, NP_000670, NP_001690, NP_068713. Weitere Gensequenzen für Säuger-Membranproteine, insbesondere der vorstehend gelisteten Membranproteine, sind durch Recherchen in der genannten Gendatenbank unschwer identifizierbar.

Gensequenzen codierend für geeignete Promotoren (Promotoren der jeweiligen heterolog exprimierten Enzyme und/oder Proteine können in einem erfindungsgemäßen Organismus jeweils gleich oder verschieden sein) sind bekannt beispielsweise aus den folgenden Acc.-No. der Gendatenbank The National Center for Biotechnology Information (NCBI): NC_001142, NC_001139, NC_001147, NC_001139, NC_001148, NC_001135, NC_001136.

Eine enzymatische Aktivität ist in einem erfindungsgemäßen Organismus gegenüber dem Wildtyp erhöht, wenn die Aktivität zumindest um 10%, vorzugsweise zumindest um 50%, höchstvorzugsweise zumindest um 1000% höher als die gleiche Aktivität im entsprechenden Wildtyp ist. Der Begriff der Erhöhung umfasst dabei auch die Anwesenheit der betreffenden enzymatischen Aktivität in einem erfindungsgemäßen Organismus, wenn der entsprechende Wildtyp keinerlei gleiche Aktivität aufweist.

Ein Enzym weist HMG-CoA-Reduktase Aktivität auf, wenn es die Reaktion von beta-Hydroxy-beta-Methylglutaryl-Coenzym-A zu Mevalonat zu katalysieren vermag.

Ein Enzym weist C24-Methyltransferase Aktivität auf, wenn es die Reaktion von Zymosterol zu Fecosterol zu katalysieren vermag.

Ein Enzym weist delta22-desaturase Aktivität auf, wenn es die Reaktion von Ergosta-5,7,24(28)-trienol zu Ergosta-5,7,22,24(28)-tetraenol zu katalysieren vermag.

Ein Enzym weist Dehydrocholesterol-delta7-Reduktase (kurz: delta7-Reduktase) Aktivität auf, wenn es die Reaktion von Cholesta-5,7,24-trien-3-ol zu Desmosterol zu katalysieren vermag.

Ein Enzym weist Dehydrocholesterol-delta24-Reduktase (kurz: delta24-Reduktase) Aktivität auf, wenn es die Reaktion von Cholesta-7-24-dien-3beta-ol zu Lathosterol, von Cholesta-5,7,24-trien-3-ol zu 7-Dehydrocholesterol, und/oder von Desmosterol zu Cholesterol zu katalysieren vermag.

Ein Enzym weist Squalen-Epoxidase Aktivität auf, wenn es die Reaktion von Squalen zu Squalenepoxid zu katalysieren vermag.

Ein Enzym weist Lanosterol-Demethylase Aktivität auf, wenn es die Reaktion von Lanosterol zu 4,4-Dimethylcholesta-8,14,24-trienol zu katalysieren vermag.

Ein Enzym weist delta7-delta8-Isomerase Aktivität auf, wenn es die Reaktion von Fecosterol zu Episterol, und/oder von Zymosterol zu Cholesta-7-24-dien-3beta-ol zu katalysieren vermag. Ein solches Enzym wird vorzugsweise bereits in dem Wildtyp des erfindungsgemäßen Organismus exprimiert. Falls der Wildtyp dieses Enzym nicht oder nicht in ausreichendem Maße exprimiert, kann der erfindungsgemäße Organismus mit einem für dieses Enzym codierenden Gen, unter der Kontrolle eines vorzugsweise konstitutiv aktiven Promotors, transformiert werden.

Ein Enzym weist delta5-Desaturase Aktivität auf, wenn es die Reaktion von Episterol zu Ergosta-5-7-24(28), von Cholesta-7-24-dien-3beta-ol zu Cholesta-5,7,24-trien-3-ol, und/oder von Lathosterol zu 7-Dehydrocholesterol zu katalysieren vermag. Ein solches Enzym wird vorzugsweise bereits in dem Wildtyp des erfindungsgemäßen Organismus exprimiert. Falls der Wildtyp dieses Enzym nicht oder nicht in ausreichendem Maße exprimiert, kann der erfindungsgemäße Organismus mit einem für dieses Enzym codierenden Gen, unter der Kontrolle eines vorzugsweise konstitutiv aktiven Promotors, transformiert werden.

Generell wird eine der vorstehend angesprochenen enzymatischen Aktivitäten dadurch gemessen, dass eine vorgegebene Menge des betreffendes Eduktes unter Zugabe einer vorgegebenen Menge des betreffenden Enzyms sowie ggf. vorgegebener Mengen der weiteren erforderlichen Reaktionspartner zu dem Produkt umgesetzt und die in einer vorgegebenen Zeitspanne gebildete Menge des Produktes bestimmt wird.

Die Bestimmung der HMG-CoA-Reduktase Aktivität, der Lanosterol-Demthylase Aktivität, der Squalen-Epoxidase Aktivität, der C24-Methyltransferase Aktivität, der delta7-delta8-Isomerase Aktivität, der delta5-Desaturase Aktivität, der delta 22-Desaturase Aktivität und der delta24-Reduktase Aktivität erfolgt beispielsweise wie in der Literaturstelle WO 03/064650 A1 beschrieben. Die Bestimmung der delta7-Reduktase Aktivität erfolgte analog.

Der Gehalt an Desmosterol und/oder Cholesterol ist in einer Membran, insbesondere einer Plasmamembran, eines erfindungsgemäßen Organismus gegenüber dem Wildtyp erhöht, wenn die Menge der betreffenden Substanz zumindest um 10%, vorzugsweise zumindest um 50%, höchstvorzugsweise zumindest um 1000% höher als die gleiche Aktivität im entsprechenden Wildtyp ist. Der Begriff der Erhöhung umfasst dabei auch die Anwesenheit der betreffenden Substanz in der Membran eines erfindungsgemäßen Organismus, wenn in der Membran eines entsprechenden Wildtyps die Substanz nicht nachweisbar ist. Die Messung der Menge erfolgt dabei, wie in den Beispielen unter den Stichworten "Aufarbeitung der Membransterole" und "Bedingungen für die Gaschromatographie (GC)" angegeben.

Eine weitere Verwendung eines erfindungsgemäßen Organismus lehrt die Herstellung eines Membranproteins eines Säugers, insbesondere eines humanen Membranproteins, vorzugsweise eines Plasmamembranproteins, und ist dadurch gekennzeichnet, dass der mit einen Gen, codierend für ein Membranprotein eines Säugers, transformierte Organismus kultiviert wird, vorzugsweise ohne Zugabe von Cholesterol und/oder Desmosterol, und dass nach Ablauf einer vorgegebenen Kultivierungsdauer das Membranprotein aus einem Kultivierungsüberstand und/oder aus dem Organismus isoliert wird.

Eine weitere Verwendung eines erfindungsgemäßen Organismus betrifft das Screenen nach an ein Membranprotein eines Säugers, insbesondere an ein humanes Membranprotein, vorzugsweise an ein Plasmamembranprotein, bindenden Substanzen, wobei der mit dem Gen für das Membranprotein transformierte Organismus oder ein Membranextrakt des transformierten Organismus, ggf. nach vorheriger Kultivierung des Organismus, mit einer vorgegebenen Substanz oder einer Mischung von vorgegebenen Substanzen kontaktiert wird, dass Veränderungen oder Abwesenheit von Veränderungen von Eigenschaften des Organismus, des Membranextraktes, und/oder die Bindung oder Nicht-Bindung einer Substanz an das Membranprotein gemessen wird, und dass bei Messung von Veränderungen und/oder Bindung die Substanz oder die Mischung von Substanzen als an das Membranprotein bindend eingestuft wird. Zu den bestimmbaren Eigenschaften des Organismus gehören die Bestimmung der Lebensfähigkeit, der quantitativen Bestimmung des Zellwachstums und der quantitativen Bestimmung vorgegebener Stoffwechselprodukte der Zellen nach Ablauf einer vorgegebenen Zeit der Kontaktierung mit einer prospektiven Substanz oder einer prospektiven Mischung von Substanzen und der Vergleich der bestimmten Größe mit derselben Größe vor der Kontaktierung. Die Bindung einer prospektiven Substanz an ein Membranprotein kann mit allen fachüblichen Bindungsessays erfolgen. Hierzu kann es vorgesehen sein, dass das Membranprotein und/oder die Substanz mit einem mittels physikalischer, physikochemischer oder chemischer Methoden detektierbares Reportermolekül kovalent verbunden ist.

Die Erfindung betrifft gemäß Anspruch 10 weiterhin ein Kit zur Durchführung eines erfindungsgemäßen Screeningverfahrens. Es enthält einen erfindungsgemäßen Organismus, optional einen zur Transformation des Organismus geeigneten Nukleinsäurekonstrukt enthaltend eine Nukleinsäure codierend für ein Membranprotein eines Säugers, insbesondere ein humanes Membranprotein, vorzugsweise ein Plasmamembranprotein, welche unter der Kontrolle eines vorzugsweise konstitutiv aktiven Promotors steht, und eine Gebrauchsanweisung zur Transformation des Organismus mit dem enthaltenen oder separat zu Verfügung gestellten Nukleinsäurekonstrukt, zur Kultivierung des Organismus vor und nach der Transformation, zur Kontaktierung des Organismus mit einer vorgegebenen Substanz oder einer vorgegebenen Mischung von Substanzen, und zur Messung einer Veränderung einer Eigenschaft des Organismus und/oder einer Bindung der Substanz oder Mischung von Substanzen an das Membranprotein.

Im Rahmen der Erfindung einsetzbar ist auch ein Nukleinsäurekonstrukt, insbesondere Plasmid oder Shuttle-Vektor, enthaltend eine Nukleinsäure oder mehrere Nukleinsäuren, gleich oder verschieden, codierend für ein Protein mit Dehydrocholesterol-delta7-Reduktase-Aktivität und/oder Dehydrocholesterol-delta24-Reduktase-Aktivität, wobei die Nukleinsäure(n) (jeweils) unter der Kontrolle eines vorzugsweise konstitutiv aktiven Promotors steht bzw. stehen. Das Nukleinsäurekonstrukt kann zusätzlich enthalten: eine Nukleinsäure oder mehrere Nukleinsäuren, gleich oder verschieden, codierend für ein Protein mit HMG-CoA-Reduktase- und/oder Squalen-Epoxidase- und/oder Lanosterol-Demethylase-Aktivität, wobei die Nukleinsäure(n) (jeweils) unter der Kontrolle eines vorzugsweise konstitutiv aktiven Promotors steht bzw. stehen.

Ein solches Nukleinsäurekonstrukt lässt sich zur Herstellung eines erfindungsgemäßen Organismus verwenden, wobei ein Vorläufer-Organismus, mit dem Nukleinsäurekonstrukt transformiert wird, wobei der Vorläufer Organismus vorzugsweise ein genetisch veränderter Organismus mit einer gegenüber dem Wildtyp reduzierten C24-Methyltransferase- und/oder delta22-Desaturase-Aktivität ist.

Schließlich betrifft die Erfindung nach Anspruch 13 ein Membranextrakt, insbesondere Plasmamembranextrakt, erhältlich aus einem erfindungsgemäßen Organismus durch Aufschluss des Organismus und Abtrennung der Membran, insbesondere der Plasmamembran. Geeignete Verfahren zum Aufschluss bzw. zur Abtrennung und Isolierung der Membran sind dem Fachmann gut bekannt.

Alle vorstehenden und nachfolgenden Erläuterungen und bevorzugte Varianten zu einem Aspekt der Erfindung finden entsprechende Anwendung bei den anderen Aspekten der Erfindung, ohne dass die jeweiligen Merkmale zu jedem Aspekt der Erfindung explizit wiederholt zu werden brauchen. Alle offenbarten Merkmale sind beliebig mit verschiedenen Aspekten der Erfindung kombinierbar, unabhängig davon in welchem Zusammenhang die Merkmale konkret offenbart sind.

Im Folgenden wird die Erfindung anhand von Beispielen näher erläutert.

### Beispiel 1: verwendete Materialien und Methoden

### 1.1: Primer

Alle Primer wurden von der Firma Metabion (Martinsried) synthetisiert.

**Tab. 1: PCR Primer zur Amplifikation. Die Erkennungssequenzen für die Restriktionsenzyme NotI und XhoI sind unterstrichen.**

| Primer | Sequenz |
|---|---|
| mSERTNotIf | 5'-ATGCGGCCGCACCATGGAGACCACACCTTTG-3' |
| mSERTXhoIr | 5'-ATCTCGAGTTACACAGCATTCATGCGG-3' |

### 1.2: Plasmide

pFlat-Vektorsystem (Veen, M., Dissertation, TU-Berlin (2002)) In den folgenden Versuchen wurden verschiedene Stämme der Hefe S. cerevisiae mit dem pFlat1- bzw. pFlat3-Vektor des pFlat-Vektorsystems transformiert (Jacobs BL, Azmitia EC. Physiol Rev (1992) 72, 165-229). Diese E. coli ↔ S. cerevisiae "Shuttle-Vektoren" enthalten eine NotI und eine XhoI Restriktionsschnittstelle in der "Multible cloning site", welche in der vorliegenden Arbeit zur Klonierung genutzt wurden. Des Weiteren tragen diese Plasmide das LEU2-(pFlat3) bzw. das URA3-Gen (pFlat1), welche als Auxotrophie-Markergene zur Selektion Plasmid-tragender Stämme auf entsprechenden Minimalmedien genutzt wurden.

### pCis-rSERT

Der eukaryotische Expressionvektor pCis beinhaltet die cDNA des Ratten-Serotonin-Transporters, welcher zwischen die Restriktionsschnittstellen XhoI und HpaI integriert wurde.

### 1.3: Stämme

### Escherichia coli K12 JM109

[recA1 endA1 gyrA96 thi hsdR17 supE44 λ⁻relA1 Δ (lac-proAB)/F' traD36 proA⁺B⁺lac^{q}lacZAM15]
(Yanisch-Perron, C., Vieira, J., Messing, J. Gene (1985) 33 (1), 103-119)

### Saccharomyces cerevisiae GRFura3

[MATa leu2-3, leu2-112, his4-519, can1, ura3Δ]
(Veen, 2002, s.o.)

### Saccharomyces cerevisiae GRFcol

[MATa tHMG1-leu2, ERG1-erg5, ERG11-erg6, his3, ura3, cyan1 ]
(Veen, 2002, s.o.)

### 1.4: Medien

### LB-Medium:

1% Trypton; 0,5% Hefe-Extrakt; 0,5% NaCl; pH 7,4

### YE-Medium:

0,5% Hefeextrakt; 2% Glucose; pH 6,3

### YNB-Medium:

0,67 % YNB ("yeast nitrogen base", Difco, Augsburg); 2% Glucose

### WMVIII:

(Lang und Looman, s.o.). Für 1 Liter Medium: 50 g Saccharose; 250 mg NH₄H₂PO₄; 2,8 NH₄Cl; 250 mg MgCl₂ x 6H₂O; 100 mg CaCl₂ x 2H₂O; 2 g KH₂PO₄; 550 mg MgSO₄ x 7H₂O; 75 mg meso-Inositol; 10 g Na-Glutamat; 1 ml 1000 x Spurenelementelösung; 4 ml 250 x Vitaminlösung

### Spurenelemente:

1000x konzentriert: 1,75 g ZnSO₄ x 7H₂O; 0,5 g FeSO₄ x 7 H₂O; 0,1 g CuSO₄ x 5 H₂O ; 0,1 g MnCl₂ x 4 H₂O ; 0,1 g NaMoO₄ x 2 H₂O für einen Liter

### Vitaminlösung:

250x konzentriert: 2,5 g Nicotinsäure; 6,25 g Pyridoxin; 2,5 g Thiamin; 0,625 g Biotin; 12,5 g Ca-Pantothenat für 1 Liter

Für Agarplatten wurde 1,5 % Agar (Serva, Heidelberg) zum Medium hinzugegeben.

### Antibiotika:

Ampicillin (Boehringer, Mannheim) 50 µg/ml

### Mediensupplemente:

Leucin (0,4 g/l); Histidin-HCl (20 mg/l)

### 1.5: Puffer und Chemikalien

### Laemmli-Puffer (4x):

0,2 M Tris-HCl pH 6,8 ; 8% SDS; 0,4% Bromphenolblau; 40% Glycerol; 400 mM DTT; 4% Triton-X

### PBS ("Phosphat buffered saline"):

150 mM NaCl; 8,4 mM Na₂HPO₄ ; 1, 6 mM KH₂PO₄, pH 7,4 PBS-T:
1 x PBS pH 7,4; 0,05 % Tween 20

### Stopper-Lösung (4x):

60% (w/v) Saccharose; 20 mM EDTA; 0,025% (w/v) Bromphenolblau TAE-Puffer: 20 mM Natriumacetat; 40 mM Tris; 2 mM EDTA; pH 8,3

### TB-1 Puffer :

100 mM NaCl ; 2 mM KCl; 1 mM CaCl_{2;} 1 mM MgCl₂; 10 mM HEPES; pH 7,5

### TE-Puffer (10x):

0,1 M Tris-HCl; 0,01 M EDTA, pH 8,0

### TED-Puffer:

10 mM Tris- HCL pH 7,6; 1 mM EDTA; 1 mM DTT

### 1.6: Methoden

### 1.6.1: Anzucht

### Aerobe Kultivierung

Die Anzucht von E. coli erfolgte in 100 ml Erlenmeyerkolben bei 37°C und 160 rpm auf einem Rundschüttler in LB-Medium. Zur Selektion Plasmid-tragender Stämme wurde das Antibiotikum Ampicillin (50 µg/ml) eingesetzt. Vorkulturen der Hefe S. cerevisiae wurden bei 30°C und 160 rpm in 100 ml Erlenmeyerkolben und Hauptkulturen in 250 ml Schikanekolben auf einem Rundschüttler angezogen. Wenn nicht ausdrücklich anders beschrieben, erfolgten sämtliche Kultivierungen der Hefe S. cerevisiae in WMVIII-Medium (C. Lang, A.C. Looman. Appl. Microbiol. Biotechnol. (1995) 44: 147-156), supplementiert mit 1 mg/ml Histidin.

### Anaerobe Kultivierung

Wenn ausdrücklich vermerkt, erfolgte die Kultivierung des Stammes S. cerevisiae GRFura3 pFlat3-rSERT bzw. pFlat3-leer unter strikt anaeroben Bedingungen, wie folgt: Eine 20 ml Vorkultur wurde, wie unter "aerobe Kultivierung" beschrieben, für 48 h angezogen. Die Hauptkultur wurde 1%ig mit dieser Vorkultur beimpft und in einem 250 ml Schikanekolben in 50 ml WMVIII-Medium, supplementiert mit 1 mg/l Histidin und 40 mg/l Cholesterol, unter strikt anaeroben Bedingungen bei 30°C und 160 rpm auf einem Rundschüttler kultiviert. Hierzu wurde der Schikanekolben in einem 2,5 l Anaeroben-Topf (Merck, Darmstadt) zusammen mit zwei Gaspacks (Anaerocult A, Merck, Darmstadt) inkubiert. Die Cholesterol-Supplementierung erfolgte mit 0,5 ml (pro 50 ml Medium) einer CholesterolLösung (2,5 g Ethanol; 2,5 g Tween 80; 20 mg Cholesterol), so dass eine Endkonzentration von 40 mg Cholesterol pro 1 Liter Medium vorlag.

### 1.6.2: Methoden der DNA-Analyse

### Restriktion

Die Restriktion der DNA (1 bis 10 µg) erfolgte in 30 µl Ansätzen, bestehend aus DNA, 3 µl des entsprechenden Puffers, 1 µl RSA (3 mg/ml) und 1 U Enzyme (NotI und XhoI, BioLabs, Schwalbach) je 1 µg eingesetzter DNA. Der Ansatz wurde bei 37°C für zwei Stunden inkubiert. Zur Restriktion von PCR-Amplifikaten wurde vier Stunden inkubiert.

### Agarose-Gelelektrophorese

Die Agarosegelelektrophorese wurde in Minigelgelapparaturen (BioRad, München) durchgeführt. Die Gele bestanden aus 1%iger Agarose in TAE-Puffer. Als Laufpuffer wurde ebenfalls TAE-Puffer verwendet. 10 µl der Probe wurden mit 3 µl einer Stopper-Lösung versetzt. Als Standard diente für Fragmente zwischen 2 kb und 13 kb λ-DNA (BioLabs, Schwalbach) restringiert mit HindIII (Bandengröße: 23,1 kb; 9,4 kb; 6,6 kb; 4,4 kb; 2,3 kb; 2,0 kb und 0,6 kb). Für kleinere Fragmente zwischen 500 bp und 3 kb wurde der Marker GeneRuler (MBI Fermentas, St. Leon-Rot) und für Fragmente zwischen 3 kb und 10 kb der Marker 1 kb DNA Ladder (New England Biolabs) verwendet. Zur Auftrennung von DNA-Fragmenten wurde eine Spannung von 100 V für 30-45 min angelegt. Danach wurde das Gel in einer Ethidiumbromidlösung (0,4 mg/l TAE-Puffer) angefärbt und unter UV-Licht (254 nm) aufgenommen.

### DNA-Fällung

DNA aus wässrigen Lösungen wurde mittels einer Fällung konzentriert. Dazu wurde die DNA-Lösung mit 1/10 Volumen 3 M Natriumacetat (pH 4,8) und einem 2,5 fachen Volumen Ethanol (96%) versetzt. Der Ansatz wurde für 30 min bei -20°C inkubiert und anschließend bei 14000 x g zentrifugiert. Das DNA Pellet wurde daraufhin noch einmal mit 70%-igem Ethanol gewaschen und bei 14000 g für 5 min zentrifugiert. Das luftgetrocknete Pellet wurde in einem gewünschten Volumen TE-Puffer resuspendiert und bei -20°C gelagert.

### Ligation

Die T4-DNA-Ligase katalysiert die Knüpfung kovalenter Phosphodiesterbindungen zwischen der 3'-Hydroxylgruppe eines Nukleotids und der 5'-Phosphatgruppe eines zweiten Nukleotids in doppelsträngiger DNA. Die zu ligierenden Fragmente wurden mittels eines DNA-Aufreinigungskits (Qiagen, Hilden) nach Angaben des Herstellers gereinigt und in einem Vektor zu Insert DNA-Verhältnis von 3:1 vereinigt und in einem Volumen von 17 µl aufgenommen. Anschließend wurden 2 µl Ligase-Puffer (MBI Fermentas, St. Leon-Rot) und 1 µl T4-Ligase (1 U/µl) hinzugegeben und der Ansatz für 2 Stunden im Fall einer "sticky-end-Ligation" bei 16°C inkubiert.

### Kompetente Zellen und Transformation von E. coli

Die Transformation von E. coli-Zellen mit Plasmid-DNA erfolgte nach der Elektroporationsmethode. Zur Präparation elektrokompetenter Zellen wurde eine Übernachtkultur 1% in 500 ml frisches LB-Medium überimpft und aerob bei 37°C bis zu einer optischen Dichte von 0,5-0,7 angezogen. Anschließend wurden die Zellen bei 5000 x g für 10 min abzentrifugiert und in 200 ml eiskaltem 10%igem Glycerol resuspendiert. Nach einem weiteren Zentrifugationsschritt bei 5000 x g für 10 min wurden die Zellen in 100 ml eiskaltem 10%igem Glycerol resuspendiert. Nach erneuter Zentrifugation bei 5000 x g für 10 min und Resuspendierung in 50 ml eiskaltem 10%igem Glycerol, wurden die Zellen für 10 min bei 5000 x g abzentrifugiert, der Überstand verworfen und das Pellet im zurückfließenden Glycerol resuspendiert, in 65µl Aliquots aufgeteilt und bei -80°C eingefroren. Zur Transformation wurden 100-200 ng Plasmid-DNA mit 30 µl der kompetenten Zellen in einem 1,5 ml Eppi gemischt und für eine Minute auf Eis inkubiert. Anschließend wurde der Ansatz in sterile, auf Eis vorgekühlte Elektroporationsküvetten überführt und die Elektroporation (1,67 kV, 25 µF, 200 Ω, 5 ms) mit einem Multiporator (Eppendorf, Hamburg) durchgeführt. Nach Pulsgebung erfolgte die sofortige Zugabe von 1 ml YE-Medium und die Überführung in ein 1,5 ml Eppi. Der Ansatz wurde nun zur Regeneration für 1 h bei 37°C inkubiert und anschließend auf LB-Agar Platten mit Ampicillin zur Selektion ausplattiert.

### Isolation von Plasmid-DNA aus E. coli (Miniprep)

Die betreffenden E. coli Stämme wurden in 5 ml LB-Medium mit 50 mg/l Ampicillin angeimpft und über Nacht bei 37°C angezogen. Die Zellen wurden anschließend bei 5000 x g zentrifugiert. Der Überstand wurde verworfen und die Plasmid-DNA mittels eines Miniprep-Kits (Qiagen, Hilden) nach Angaben des Herstellers aufgearbeitet.

### Polymerase-Ketten-Reaktion (PCR)

Durch die Polymerase-Ketten-Reaktion ist es möglich, mittels spezifischer Primer DNA-Fragmente aus isolierter DNA oder direkt aus ganzen E. coli Zellen zu amplifizieren. Standardmäßig wurde für eine PCR-Reaktion 100 ng Template-DNA, gelöst in 1 µl H₂O oder eine E. coli Kolonie, mit einem Zahnstocher in ein PCR-Tube überführt (Kolonie-PCR), eingesetzt. Neben dem Template bestand ein Reaktionsansatz aus 4 µl dNTP-Mix (2,5 mM), 5 µl 10x Reaktionspuffer, je 2 µl Primer (forward und revers), 0,2 µl (1 U) Taq-Polymerase (Promega, Mannheim) oder Takara-Polymerase (Takara, Tokio, Japan) und H₂O in einem Gesamtvolumen von 50 µl. Die PCR wurde in einem Thermocycler (Biorad, München) je nach Ansatz durchgeführt. Zur Analyse wurden 10 µl des PCR-Ansatzes auf einem Agarose-Gel aufgetrennt oder zur weiteren Verwendung mittels eines PCR-Aufreinigungskits (Qiagen, Hilden) gereinigt und auf ein Volumen von 30 µl konzentriert.

### Hefetransformation

Für die Hefetransformation wurde eine Vorkultur in 20 ml YE-Medium über Nacht kultiviert. Ein 50 ml Hauptkultur wurde auf eine optische Dichte OD₆₀₀ von 0,3 angeimpft und für etwa 4 Stunden kultiviert. Bei einer erreichten OD₆₀₀ von 0,8- 1,0 wurden die Zellen bei 4000 g für 10 min zentrifugiert und das Zellpellet mit 10 ml sterilem H₂O gewaschen. Anschließend wurden die Zellen in 1,5 ml H₂O aufgenommen und in ein Eppi überführt. Die Zellen wurden zentrifugiert (4000 g, 2 min) und in 1 ml Lithiumacetat-Lösung (100 mM LiAc in TE-Puffer) resuspendiert, zentrifugiert (4000 x g, 2 min) und nochmals mit 1 ml Lithiumacetat-Lösung gewaschen. Anschließend wurden die Zellen in 200 µl Lithiumacetat-Lösung aufgenommen (kompetente Zellen). Für einen Transformationsansatz wurden 40 µl kompetente Zellen, 10 µl Heringssperma-DNA (10 mg/ml), 230 µl PEG-Lösung (40% PEG 4000 in 0,1 M Lithiumacetat in 1 x TE pH 8,0) und 1-20 µg zu transformierende DNA eingesetzt. Dieser Ansatz wurde für 30 min bei 30°C inkubiert und anschließend einem Hitzschock bei 42°C für 8 min ausgesetzt. Nach Abkühlen auf Raumtemperatur wurden 800 µl H₂O zugesetzt und der Ansatz bei 7000 x g für 20 s zentrifugiert.

Anschließend wurden die Zellen direkt auf entsprechenden Selektionsmedien mit Agar ausplattiert und für 3 bis 5 Tage bei 30°C inkubiert. Im Falle der in Kapitel 4.2.2 erwähnten Doppeltransformation, also der gleichzeitigen Transformation mit 2 Vektoren, enthielt der Transformationsansatz etwa 30 µg jedes Vektors. Dazu wurde die Vektor-DNA gefällt und in 10 µl H₂O aufgenommen. 40 µl kompetente Zellen wurden zugegeben und dieser Ansatz 30 min bei 37°C inkubiert, bevor die PEG-Lösung und die Heringssperma-DNA zugegeben wurde. Danach wurde wie oben beschrieben verfahren.

### 1.6.3: Sterolanalytik

### Kultivierungsbedingungen für die Sterolanalyse

Für die Sterolanalyse wurden Hefekulturen nach folgendem Schema geführt: Eine 20 ml Vorkultur wurde mit 50 µl einer Gefrierkultur angeimpft und für 2 Tage kultiviert. Die Hauptkultur wurde in 250 ml Schikanekolben mit 50 ml Medium (WMVIII-Medium, supplementiert mit 1 mg/ml Histidin) auf einem Rundschüttler bei 160 rpm und 30°C für 1 bzw. 3 Tage geführt. Angeimpft wurde die Hauptkultur mit 1% der Vorkultur.

### Bestimmung der Trockensubstanz

Zur Bestimmung der Trockensubstanz wurden 3 mal 6 ml Kulturvolumen in vorher ausgewogene 15 ml Greiner-Röhrchen überführt. Die Zellen wurden bei 3500 g für 5 min abzentrifugiert und mit 10 ml H₂O gewaschen. Anschließend wurde das Zellpellet in einem Trockenschrank für 12 Stunden bei 80°C getrocknet. Die Proben kühlten im Exikator ab, bevor die Wägung erfolgte.

### Aufarbeitung der Gesamtsterole

Die zu untersuchenden Zellen wurden geerntet und mit 40 ml H₂O gewaschen. Anschließend wurde die optische Dichte OD₆₀₀ bestimmt und ein Hefepellet, das 125 OD₆₀₀ entspricht, in 5 ml 0,5 N HCl-Lösung aufgenommen. Die Suspension wurde dann in einem 50 ml Falkon-Röhrchen für 20 min bei 100°C gekocht. Die derart labilisierten Zellen wurden anschließend auf Eis abgekühlt und der Lösung 3 g KOH zugegeben. Nach Auflösen des KOH wurden 12,5 ml einer 0,25 g/l methanolischen Pyrogallol-Lösung zugesetzt. Als Extraktionsstandard wurden zusätzlich 10 µl Cholesterollösung (10 mg/ml) zugefügt. Das Verseifungsgemisch wurde für 1 h 45 min bei 70°C inkubiert. Nach Abkühlung dieses Ansatzes auf Raumtemperatur wurden die Sterole mit 2 x 20 ml n-Hexan extrahiert. Die kombinierten n-Hexan-Phasen wurden am Rotationsverdampfer eingeengt und der Rückstand mit 2 x 1 ml Chloroform mit 0,2 g/l Nonadekan als internem Standard gelöst und in Braunglasflaschen überführt. Die Sterol-Extrakte wurden bis zur Analyse bei -20°C gelagert.

### Aufarbeitung der Membransterole

Zur Präparation der Proben für die Membransterolanalytik mussten zuerst die Plasmamembranen der zu untersuchenden Stämme der Hefe S. cerevisiae isoliert werden. Dies erfolgte nach der Methode von Serrano (Serrano et al., Methods Enzymol., 157:533-544 (1988) mit einigen Modifikationen, welche im Folgenden kurz beschrieben wird: Die Zellen wurden nach der Ernte und einem Waschschritt mit H₂O in 1 ml 1 M Tris (pH 8), 200 µl 0.5 M EDTA and 100 µl 200 mM PMSF aufgenommen. Anschließend erfolgte der Aufschluss mit Glasperlen. Nach einem Zentrifugationsschritt wurde der Überstand abgenommen, die Glasperlen mit TED-Puffer gewaschen und die Überstände in einem SS34-Röhrchen vereinigt. Größere Membranvesikel und die Plasmamembran wurden nun bei 20000 g für 30 min pelletiert und das Pellet in 4 ml 20% Glycerol in TED-Puffer mit 200 µl 0,2 M PMSF aufgenommen und mit einem Dounce-Homogenisator homogenisiert. 3 ml dieses Homogenats wurden auf einen diskontinuierlichen Gradienten, aus 6 ml 53% (w/w) und 3 ml 43% (w/w) Saccharose in einem Beckman Ultrazentrifugen-Röhrchen, gegeben. Die Zentrifugation erfolgte für 6 h bei 100.000 g in einem Beckman SW41-Ti Rotor. Die aufgereinigte Plasmamembran konnte mit einer Pasteurpipette aus der Interphase abgenommen werden und wurde in ein neues Beckman-Röhrchen überführt, welches mit H₂O aufgefüllt wurde. Die Plasmamembran wurde anschließend bei 80000 g für 30 min pelletiert und das Pellet in 2 ml TED-Puffer resuspendiert und bei -20°C gelagert. Zur Extraktion der Sterole wurde 1 ml der Plasmamembran-Suspension mit 500 µl Chloroform versetzt und 1 min gevortexed. Es folgte die Phasentrennung bei 13000 rpm für 15 min. 450 µl der unteren organischen Phase wurden nun in ein GC-Röhrchen überführt und 50 µl N-Methyl-N-trimethylsilyltrifluoracetamid (MSTFA, Sigma, München) zugegeben. Die Derivatisierung der Sterole erfolgte für 1 h bei 80°C. Die fertigen Proben konnten nun gaschromatographisch analysiert werden.

### Bedingungen für die Gaschromatographie (GC)

Die GC-Analytik wurde mit einem Agilent 6890N Gaschromatographen (Agilent, Waldbronn), ausgestattet mit einem Autosampler Agilent 7683B, durchgeführt. Als Detektor wurde ein Agilent 5975 VL Massenspektrometer verwendet. Es wurden folgende Bedingungen gewählt: Als Säule diente eine 30m lange HP-5MS Säule (J&W Scientific, USA) mit einem Innendurchmesser von 0,25 mm und einer Filmdicke von 0,25µm. Helium diente als mobile Phase. Das GC/MS System wurde mit einem Temperaturprogramm (100°C 0,5 min, 50°C/min bis 270°C, 270°C für 10 min, 5°C/min bis 290°C, 290°C für 4min, 5°C/min bis 325°C, 325°C für 20min, 5°C/min bis 340°C, 340°C für 2min) im Splitless-Modus gefahren. Die Injektortemperatur betrug 280°C, die des Detektors 150°C. Zur besseren Trennung und der Erhöhung der Flüchtigkeit von Sterolintermediaten, wurden die Sterolproben mit MSTFA für eine Stunde bei 80°C im Trockenschrank derivatisiert. Die Bestimmung der molaren Massen der untersuchten Sterolintermediate, sowie deren Identifizierung anhand des Ionisierungsmusters, erfolgte mit dem Agilent 5975 VL Massenspektrometer. Das Injektionsvolumen der Proben betrug 2 µl.

### Beispiel 2: Ergebnisse

Im Jahre 2002 gelang es die Biosynthese von Cholesta-5,7,24-trien-3-ol in der Hefe S.cerevisiae zu etablieren (Veen, 2002, s.o.). Dazu wurde ein frei erhältlicher Laborstamm GRF18 wie folgend dargestellt mit gentechnischen Methoden modifiziert.

| **Stamm** | | **Beschreibung** |
|---|---|---|
| **GRF18** | **↓** | ***leu2*, *his3*** |
| **GRF*ura3*** | **↓** | ***leu2, his3, ura3*** |
| **GRF*tH3*** | **↓** | ***tHMG1-leu2, his3, ura3*** |
| ***GRFtH3E1e5*** | **↓** | ***tHMG1-leu2, ERG1-erg5, his3, ura3*** |
| ***GRFtH1E1E11e5e6*** | | ***tHMG1-leu2, ERG1-erg5, ERG11-erg6, his3, ura3*** |

Wie zu sehen ist, wurde zunächst das URA3-Gen deletiert und das tHMG1-Gen am Genlocus LEU2 integriert. Das tHMG1-Gen (t = truncated, verkürzt) kodiert für eine verkürzte HMG-CoA-Reduktase, welche nur noch aus der katalytischen Untereinheit des Proteins besteht und somit nicht mehr der Feedback-Hemmung durch Sterolintermediate unterliegt. Die Transkription des tHMG1-Gens wird durch den konstitutiven ADH1-Promotor kontrolliert. Durch die Analyse einer systematischen Überexpression und transkriptionellen Deregulation aller Gene des Postsqualenbiosyntheseweges einzeln und in Kombinationen im HMG1-deregulierten Stamm, konnten zwei weitere Hauptregulationspunkte bzw. Engpässe im Postsqualenweg identifiziert werden. Diese Engpässe konnten durch die Überexpression der Squalen-Epoxidase (ERG1) und der Lanosterol-Demethylase (ERG11) überwunden werden. Dazu wurden die Gene ERG1 und ERG11 in einer transkriptionell deregulierten Form unter der Kontrolle eines konstitutiven ADH1-Promotors in das Hefegenom integriert, und zwar mittels homologer Rekombination an den Loci der Gene ERG5 und ERG6, welche dadurch deletiert wurden (Veen, 2002, s.o.). So wurden in nur 3 Transformationsschritten 5 genetische Veränderungen in den Hefestamm eingebracht, durch welche eine Beseitigung der Engpässe im Ergosterol-Syntheseweg, sowie die Etablierung der Synthese von Cholesta-5,7,24-trien-3-ol gelungen ist. Zusätzlich zeigte sich, dass der resultierende Hefestamm GRFtH1E1E11e5e6 (GRFcol) Post-Squalen-Sterolintermediate in weit größerer Menge als der Wildtypstamm GRFura3 produziert.

In einem weiteren Schritt wurde in diesem Stamm mittels des Shuttle-Vektors pFlat1, erfindungsgemäß eine Dehydrocholesterol-delta7-Reduktase (bekannt aus der Literaturstelle US-5,759,801) zur Überexpression gebracht. Dazu wurde die cDNA für die Dehydrocholesterol-delta7-Reduktase (DHCR7) aus Arabidopsis thaliana zwischen den konstitutiven ADH1-Promotor und den Tryptophan-Terminator (TRP1) des Vektors integriert und in die Hefe transformiert.

Der resultierende Hefestamm wurde als GRFcol pFlat1-DHCR7 bezeichnet.

### Die gaschromatographische Analyse der

Gesamtsterolzusammensetzung ist folgend dargestellt. Tabelle 2 zeigt die Zusammensetzung der Haupsterole in der Hefe GRFcol nach der Transformation mit dem Vektor pFLAT1-DHCR7, welcher die Dehydrocholesterol-Δ7-Reduktase aus Arabidopsis thaliana und/oder pFlat3-DHCR24, welcher die Dehydrocholesterol-Δ24-Reduktase aus Xenopus laevis beinhaltet. Die Werte stammen aus einer GC/MS-Analyse und verstehen sich als prozentuale Anteile. Man erkennt, dass die Überexpression der DHCR7 zu einer teilweisen Umwandlung des Cholesta-5,7,24-trienol in Cholesta-5,24-Dienol (Desmosterol), einer direkten Cholesterol-Vorstufe, führte. Der Anteil dieses Sterols am Gesamtsterolgehalt der Hefe lag bei ca. 40-50%. Somit war Desmosterol neben Zymosterol das Hauptsterol in der Hefe. Die Untersuchung der Plasmamembran ergab, dass Desmosterol mit einem Anteil von ca. 60% das Hauptmembransterol war (Daten nicht gezeigt).

**Tabelle 2: Prozentualer Anteil des jeweiligen Sterols in den angegebenen Stämmen (Gesamtsterole)**

| Stamm | Cholesterol | Desmos terol | Zymos terol | Cholesta-7,24-Dienol | Ergos terol | Cholesta-5,7,24-Trienol | Squalen | 7-Dehydro cholest erol |
|---|---|---|---|---|---|---|---|---|
| GRFcol pFlat1 pFlat3 | 0,0 | 0,0 | 14,5 | 14,0 | 5,3 | 25,4 | 40,7 | 0,0 |
| GRFcol pFlat1-DHCR7 pFlat3 | 0,0 | 47,3 | 8,7 | 5,5 | 4,2 | 13,1 | 21,3 | 0,0 |
| GRFcol pFlat1-DHCR7 pFlat3-DHCR24 X.laevis | 3,2 | 41,7 | 6,6 | 4,8 | 5,7 | 8,9 | 22,1 | 7,0 |

Nach der Etablierung der Desmosterol-Biosynthese wurde mittels des Shuttle-Vektors pFlat3 eine Dehydrocholesterol-Δ24-reduktase in der Hefe zusätzlich zu DHCR7 exprimiert. Dazu wurde die cDNA für die Dehydrocholesterol-Δ24-reduktase (DHCR24) aus Xenupus laevis zwischen den konstitutiven ADH1-Promotor und den Tryptophan-Terminator (TRP1) des pFlat3 Vektors integriert und in die Hefe eingebracht. Der resultierende Stamm wurde als GRFcol pFlat1-DHCR7 pFlat3-DHCR24 bezeichnet. Die Gaschromatographische Analyse der Gesamtsterolzusammensetzung dieses Stammes zeigte, daß die Überexpression der DHCR24 zur Synthese von Cholesterol und 7-Dehydrocholesterol führte. Der Anteil von Cholesterol und 7-Dehydrocholesterol lag bei ca. 3,2 bzw. 7,0 %. (Tabelle 1)

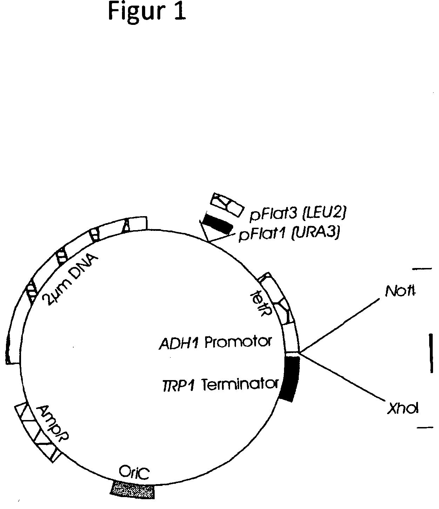

Zur Überprüfung eines Einflusses von Desmosterol auf die heterologe Expression eines Membranproteins, wurde der Serotonintransporter aus der Ratte in dem Stamm GRFcol pFlat1-DHCR7 überexprimiert. Dazu wurde die rSERT-cDNA zwischen den konstitutiven ADH1-Promotor (C. Lang, A.C. Looman, Appl. Microbiol. Biotechnol. 44: 147-156 (1995) und den Tryptophan-Terminator (TRP1) des pFlat3-Vektors integriert. Die Integration erfolgt über eine NotI und eine XhoI Restriktionsschnittstelle in der "multiple-cloning-site" dieses E. coli / S. cerevisiae "Shuttle-Vektors". Das pFlat-Vektor System ist schematisch in Figur 1 dargestellt. Sie zeigt eine schematische Darstellung der episomalen Hefeexpressionsplasmide pFlat1 und pFlat3. Das Plasmid pFlat1 trägt ein URA3-Gen und pFlat3 ein LEU2-Gen zur Selektion Plasmid-tragender Stämme. Beide Plasmide enthalten den konstitutiven ADH1-Promotor und den TRP1-Terminator, sowie den größten Teil der 2 µm DNA zur Replikation. Die Klonierung der rSERT-cDNA zwischen die Restriktionsschnittstellen NotI und XhoI der multiple-cloning-site ist schematisch dargestellt.

Nach der Erstellung des Vektors pFlat3 rSERT wurde der Stamm GRFcol pFlat1-DHCR7 sowie der Wildtypstamm GRF18mit dem Plasmid transformiert und der Serotoninrezeptor zur Expression gebracht. Die funktionelle Expression des rSERT in den zu untersuchenden Hefestämmen wurde durch Bindung von [³H] Citalopram an Spheroblasten überprüft. Serotonin-Antagonisten, wie Citalopram, binden ausschließlich an korrekt gefaltete und aktive, also an funktionell exprimierte SERT Moleküle. Citalopram bindet mit hoher Affinität an dieselbe Stelle des Serotonin-Transporters wie das eigentliche Substrat, Serotonin. Ein Indiz hierfür ist, daß Citalopram die maximale Anzahl der Bindungstellen reduziert, jedoch nicht die Affinität des Serotonins zum SERT senkt. Es ist bekannt, dass [³H] Citalopram nur an den rSERT bindet, wenn Cholesterol in der ihn umgebenden Membran vorhanden ist. Ergosterol konnte Cholesterol in dieser Studie nicht ersetzen. Ob dies mit den Sterolen Cholesta-5,7,24-trienol und Desmosterol gelingt, welche der Struktur von Cholesterol ähnlicher sind, sollte in einem Experimenten untersucht werden.

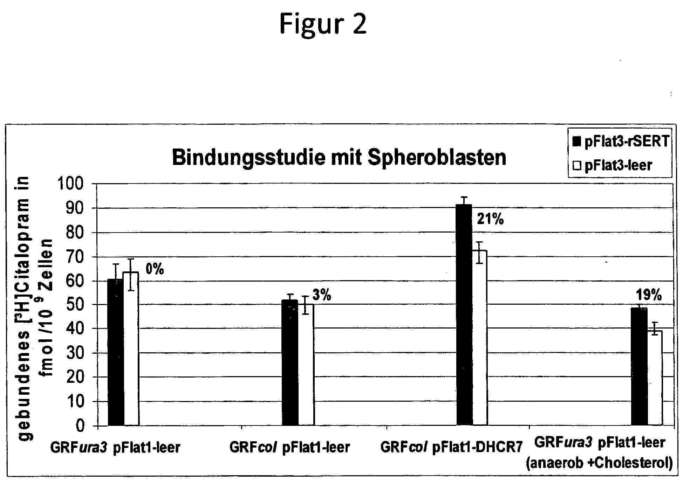

Um die funktionelle Expression des rSERT in der Plasmamembran der zu untersuchenden Hefestämme zu überprüfen, wurde eine [³H] Citalopram Bindungsstudie mit Spheroblasten dieser Stämme durchgeführt. Figur 2 zeigt die Ergebnisse dieser Studie. Dargestellt ist eine Scintillatiohszählung des gebundenen [³H] Citalopram in fmol pro 10⁹ Zellen. Spheroblasten der Stämme GRFura3 pFlat1-leer, pFlat3-rSERT; GRFcol pFlat-leer, pFlat3-rSERT; GRFcol pFlat1-DHCR7, pFlat3-rSERT; GRFura3 pFlat1-leer, pFlat3-rSERT anaerob kultiviert mit Cholesterolsupplementierung und der entsprechenden Kontrollstämme (um die unspezifische Bindung zu bestimmen, tragen den pFlat3-leer Vektor) wurden mit 2 nM [³H] Citalopram inkubiert. Die Prozentangaben repräsentieren die spezifische Bindung an der Gesamtbindung. Die Balkenhöhe repräsentiert den Mittelwert aus drei unabhängigen Experimenten. Die Fehlerbalken geben den größten und den geringsten Messwert an.

Die Figur 2 zeigt, dass die unspezifische Bindung bei GRFcol-DHCR7 am höchsten und bei GRFura3 anaerob + Cholesterol am geringsten ist.

Ein signifikanter Unterschied zwischen spezifischer und unspezifischer Bindung an Sheroblasten von GRFura3 und GRFcol, und somit eine funktionelle Expression des rSERT in diesen Stämmen, ist aus den Ergebnissen in Figur 2 nicht ersichtlich. Überraschend ist aber, dass neben GRFura3 anaerob mit Cholesterol, auch bei GRFcol-DHCR7 eine spezifische Bindung des [³H] Citalopram von etwa 20% nachweisbar ist. Dies entspricht einer spezifischen Bindung von durchschnittlich 6 Molekülen [³H] Citalopram bzw. der funktionellen Expression von 6 Molekülen rSERT pro Zelle des Stammes GRFura3 anaerob mit Cholesterol. Bei GRFcol-DHCR7 entspricht es 11 Molekülen. Die Differenz von 5 Molekülen kann durch den unterschiedlichen Expressionslevel des SERT in den beiden Stämmen erklärt werden. Eine Analyse der Expressionslevel per Western Blot (Daten nicht gezeigt) zeigte, dass die Expression in GRFcol-DHCR7 größer ist, als in GRFura3 anaerob mit Cholesterol.

Die spezifische Bindung beträgt sowohl in GRFura3 anaerob mit Cholesterol, als auch in GRFcol-DHCR7 etwa 20%. Desmosterol scheint also das Fehlen von Cholesterol, bezüglich der Funktionalität des SERT, vollständig zu kompensieren. Die Ergebnisse der Bindungsstudie mit Spheroblasten deuten auf eine funktionelle Expression des rSERT in GRFcol-DHCR7 hin.

Bei Spheroblasten des Stammes GRFura3 pFlat3-rSERT anaerob mit Cholesterol, konnte eine spezifische Bindung von [³H] Citalopram nachgewiesen werden. Es ist also davon auszugehen, dass der rSERT in diesem Stamm unter diesen Kultivierungsbedingungen funktionell exprimiert wird. Da mit Sheroblasten gearbeitet wurde, ist auch der Nachweis für die Expression des rSERT in der Plasmamembran gelungen. Die Ergebnisse in Figur 2 verdeutlicht, dass Ergosterol und Cholesta-5,7,24-trienol das Fehlen von Cholesterol nicht kompensieren können, und für eine funktionelle Expression des SERT Cholesterol bzw. Desmosterol in der Membran nötig ist.

## Patentansprüche

1. Isolierter genetisch veränderter lebender nicht-Säuger-Organismus, mit einer gegenüber dem Wildtyp erhöhten HMG-CoA-Reduktase Aktivität, und mit einer gegenüber dem Wildtyp reduzierten C24-Methyltransferase- und/oder delta22-Desaturase-Aktivität,
**dadurch gekennzeichnet,**
**dass** der Organismus eine gegenüber dem Wildtyp erhöhte Dehydrocholesterol-delta7-Reduktase-Aktivität aufweist und
**dass** der Organismus zusätzlich transformiert ist mit einem für ein Membranprotein eines Säugers codierenden Gen, unter Kontrolle eines Promotors.

2. Organismus nach Anspruch 1, **dadurch gekennzeichnet, dass** der Organismus zusätzlich eine gegenüber dem Wildtyp erhöhte Dehydrocholesterol-delta24-Reduktase-Aktivität aufweist.

3. Organismus nach einem der Ansprüche 1 oder 2, **dadurch gekennzeichnet, dass** der Organismus eine gegenüber dem Wildtyp erhöhte Squalen-Epoxidase- und/oder Lanosterol-Demethylase-Aktivität aufweist.

4. Organismus nach einem der Ansprüche 1 bis 3, **dadurch gekennzeichnet, dass** der Organismus ein prokaryotischer Organismus, insbesondere ausgewählt aus der Gruppe bestehend aus Bakterien der Gattungen Bacillus, Escherichia, Lactobacillus, Corynebacterium, Acetobacter, Acinetobacter und Pseudomonas oder ein eukaryotischer Organismus, insbesondere ausgewählt aus der Gruppe bestehend aus Algen, Hefen, Pilze, Insektenzellen, Moose und Pflanzen ist.

5. Mikroorganismus, nach Anspruch 4, **dadurch gekennzeichnet, dass** die Hefe ausgewählt ist aus der Gruppe bestehend aus Saccharomyces cerevisiae, Saccharomyces delbrückii, Saccharomyces italicus, Saccharomyces ellipsoideus, Saccharomyces fermentati, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum und Saccharomycodes ludwigii, sowie Hefen der Gattung Kluyveromyces wie K. lactis K. marxianus var. marxianus, K. thermotolerans, sowie Hefen der Gattung Candida wie Candida utilis, Candida tropicalis, Candida albicans, Candida lipolytica und Candida versatilis, sowie Hefen der Gattung Pichia wie Pichia stipidis, Piachia pastoris und Pichia sorbitophila, sowie Hefen der Gattungen Cryptococcus, Debaromyces, Hansenula, Saccharomycecopsis, Saccharomycodes, Schizosaccharomyces, Wickerhamia, Debayomyces, Hanseniaspora, Kloeckera, Zygosaccharomyces, Ogataea, Kuraishia, Komagataella, Metschnikowia, Williopsis, Nakazawaea, Cryptococcus, Torulaspora, Bullera, Rhodotorula, Willopsis und Sporobolomyces.

6. Organismus nach einem der Ansprüche 1 bis 5, **dadurch gekennzeichnet, dass** der Organismus, insbesondere die Membran, insbesondere die Plasmamembran, des Organismus einem gegenüber dem Wildtyp erhöhten Gehalt an Desmosterol und/oder Cholesterol und/oder 7-Dehydrocholesterol und/oder Lathosterol aufweisen.

7. Organismus nach einem der Ansprüche 1 bis 6, **dadurch gekennzeichnet, dass** das für ein Membranprotein eines Säugers codierende Gen ein für ein humanes Membranprotein, vorzugsweise ein Plasmamembranprotein, codierendes Gen ist, und dass dieses Gen unter Kontrolle eines konstitutiv aktiven Promotors steht.

8. Verwendung eines Organismus nach einem der Ansprüche 1 bis 7 zur Herstellung eines Membranproteins eines Säugers, insbesondere eines humanen Membranproteins, vorzugsweise eines Plasmamembranproteins, **dadurch gekennzeichnet, dass** der transformierte Organismus kultiviert wird, vorzugsweise ohne Zugabe von Cholesterol und/oder Desmosterol, und dass nach Ablauf einer vorgegebenen Kultivierungsdauer das Membranprotein aus einem Kultivierungsüberstand und/oder aus dem Organismus isoliert wird.

9. Verwendung eines Organismus nach einem der Ansprüche 1 bis 7 zum Screenen nach an ein Membranprotein eines Säugers, insbesondere an ein humanes Membranprotein, vorzugsweise an ein Plasmamembranprotein, bindenden Substanzen, **dadurch gekennzeichnet, dass** der Organismus oder ein Membranextrakt des Organismus, nach vorheriger Kultivierung des Organismus, mit einer vorgegebenen Substanz oder einer Mischung von vorgegebenen Substanzen kontaktiert wird, dass Veränderungen oder Abwesenheit von Veränderungen von Eigenschaften des Organismus, des Membranextraktes, und/oder die Bindung oder Nicht-Bindung einer Substanz an das Membranprotein gemessen wird, und dass bei Messung von Veränderungen und/oder Bindung die Substanz oder die Mischung von Substanzen als an das Membranprotein bindend eingestuft wird.

10. Kit zur Durchführung eines Verfahrens nach Anspruch 9, mit einem genetisch veränderten lebenden nicht-Säuger-Organismus, mit einer gegenüber dem Wildtyp erhöhten HMG-CoA-Reduktase Aktivität, mit einer gegenüber dem Wildtyp reduzierten C24-Methyltransferase- und/oder delta22-Desaturase-Aktivität, und mit einer gegenüber dem Wildtyp erhöhte Dehydrocholesterol-delta7-Reduktase-Aktivität, mit einem zur Transformation des Organismus geeigneten Nukleinsäurekonstrukt enthaltend eine Nukleinsäure codierend für ein Membranprotein eines Säugers, insbesondere ein humanes Membranprotein, vorzugsweise ein Plasmamembranprotein, welche unter der Kontrolle eines vorzugsweise konstitutiv aktiven Promotors steht, und mit einer Gebrauchsanweisung zur Transformation des Organismus mit dem Nukleinsäurekönstrukt, zur Kultivierung des Organismus vor und nach der Transformation, zur Kontaktierung des Organismus mit einer vorgegebenen Substanz oder einer vorgegebenen Mischung von Substanzen, und zur Messung einer Veränderung einer Eigenschaft des Organismus und/oder einer Bindung der Substanz oder Mischung von Substanzen an das Membranprotein.

11. Verwendung eines Nukleinsäurekonstrukts, insbesondere Plasmid oder Shuttle-Vektors, enthaltend eine Nukleinsäure oder mehrere Nukleinsäuren, gleich oder verschieden, codierend für ein Protein mit Dehydrocholesterol-delta7-Reduktase-Aktivität und/oder Dehydrocholesterol-delta24-Reduktase-Aktivität, wobei die Nukleinsäure(n) (jeweils) unter der Kontrolle eines vorzugsweise konstitutiv aktiven Promotors steht bzw. stehen zur Herstellung eines Organismus für ein Kit gemäß Anspruch 10, wobei ein Vorläufer-Organismus mit dem Nukleinsäurekonstrukt, das Nukleinsäurekonstrukt enthaltend eine Nukleinsäure codierend für ein Membranprotein eines Säugers, insbesondere ein humanes Membranprotein, vorzugsweise ein Plasmamembranprotein, welche unter der Kontrolle eines vorzugsweise konstitutiv aktiven Promotors steht, transformiert wird, wobei der Vorläufer Organismus vorzugsweise ein genetisch veränderter Organismus mit einer gegenüber dem Wildtyp reduzierten C24-Methyltransferase- und/oder delta22-Desaturase-Aktivität ist.

12. Verwendung nach Anspruch 11, das Nukleinsäurekonstrukt zusätzlich enthaltend eine Nukleinsäure oder mehrere Nukleinsäuren, gleich oder verschieden codierend für ein Protein mit HMG-CoA-Reduktase- und/oder Squalen-Epoxidase- und/oder Lanosterol-Demethylase-Aktivität, wobei die Nukleinsäure(n) (jeweils) unter der Kontrolle eines vorzugsweise konstitutiv aktiven Promotors steht bzw. stehen.

13. Membranextrakt, insbesondere Plasmamembranextrakt, erhältlich aus einem Organismus nach einem der Ansprüche 1 bis 7 durch Aufschluss des Organismus und Abtrennung der Membran, insbesondere der Plasmamembran, enthaltend Desmosterol und/oder Cholesterol und/oder 7-Dehydrocholesterol und/oder Lathosterol, sowie ein Membranprotein eines Säugers, erhalten aus dem für ein Membranprotein eines Säugers codierenden Gen.

## Claims

1. An isolated genetically modified living non-mammalian organism, with an increased HMG-CoA reductase activity as compared to the wild-type, and with a reduced C24-methyltransferase and/or delta22-desaturase activity as compared to the wild-type,
**characterized by**
that the organism has an increased dehydrocholesterol-delta7-reductase activity as compared to the wild-type, and
that the organism further is transformed with a gene encoding a membrane protein of a mammal, under control of a promoter.

2. The organism according to claim 1, **characterized by** that the organism further has an increased dehydrocholesterol-delta24-reductase activity as compared to the wild-type.

3. The organism according to one of claims 1 or 2, **characterized by** that the organism has an increased squalene epoxidase and/or lanosterol demethylase activity as compared to the wild-type.

4. The organism according to one of claims 1 to 3, **characterized by** that the organism is a prokaryotic organism, in particular selected from the group consisting of bacteria of the genera Bacillus, Escherichia, Lactobacillus, Corynebacterium, Acetobacter, Acinetobacter and Pseudomonas or a eukaryotic organism, in particular selected from the group consisting of algae, yeasts, fungi, insect cells, mosses, and plants.

5. The microorganism according to claim 4, **characterized by** that the yeast is selected from the group consisting of Saccharomyces cerevisiae, Saccharomyces delbrückii, Saccharomyces italicus, Saccharomyces ellipsoideus, Saccharomyces fermentati, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum, and Saccharomycodes ludwigii, as well as yeasts of the genus Kluyveromyces such as K. lactis K. marxianus var. marxianus, K. thermotolerans, as well as yeasts of the genus Candida such as Candida utilis, Candida tropicalis, Candida albicans, Candida lipolytica, and Candida versatilis, as well as yeasts of the genus Pichia such as Pichia stipidis, Piachia pastoris, and Pichia sorbitophila, as well as yeasts of the genera Cryptococcus, Debaromyces, Hansenula, Saccharomycecopsis, Saccharomycodes, Schizosaccharomyces, Wickerhamia, Debayomyces, Hanseniaspora, Kloeckera, Zygosaccharomyces, Ogataea, Kuraishia, Komagataella, Metschnikowia, Williopsis, Nakazawaea, Cryptococcus, Torulaspora, Bullera, Rhodotorula, Willopsis, and Sporobolomyces.

6. The organism according to one of claims 1 to 5, **characterized by** that the organism, in particular the membrane, in particular the plasma membrane, of the organism has an increased content of desmosterol and/or cholesterol and/or 7-dehydrocholesterol and/or lathosterol as compared to the wild-type.

7. The organism according to one of claims 1 to 6, **characterized by** that the gene encoding a membrane protein of a mammal is a gene encoding a human membrane protein, preferably a plasma membrane protein, and that this gene is under control of a constitutively active promoter.

8. A use of an organism according to one of claims 1 to 7 for making a membrane protein of a mammal, in particular a human membrane protein, preferably a plasma membrane protein, **characterized by** that the transformed organism is cultured, preferably without addition of cholesterol and/or desmosterol, and that after expiry of a predetermined culture period the membrane protein is isolated from a culture supernatant and/or from the organism.

9. A use of an organism according to one of claims 1 to 7 for screening for substances binding to a membrane protein of a mammal, in particular to a human membrane protein, preferably to a plasma membrane protein, **characterized by** that the organism or a membrane extract of the organism, after previous culture of the organism, is contacted with a predetermined substance or a mixture of predetermined substances, that alterations or absence of alterations of properties of the organism, of the membrane extract, and/or the binding or non-binding of a substance to the membrane protein is measured, and that when alterations and/or binding are measured, the substance or the mixture of substances is classified as binding to the membrane protein.

10. A kit for carrying-out a method according to claim 9, with a genetically modified living non-mammalian organism, with an increased HMG-CoA reductase activity as compared to the wild-type, with a reduced C24-methyltransferase and/or delta22-desaturase activity as compared to the wild-type, and with an increased dehydrocholesterol-delta7-reductase activity as compared to the wild-type, with a nucleic acid construct suitable for transformation of the organism and containing a nucleic acid encoding a membrane protein of a mammal, in particular a human membrane protein, preferably a plasma membrane protein, which is under control of a preferably constitutively active promoter, and with instructions of use for transforming the organism with the nucleic acid construct, for culturing the organism before and after the transformation, for contacting the organism with a predetermined substance or a predetermined mixture of substances, and for measuring an alteration of a property of the organism and/or of a binding of the substance or mixture of substances to the membrane protein.

11. A use of a nucleic acid construct, in particular a plasmid or shuttle vector, comprising a nucleic acid or a plurality of nucleic acids, identical or different, encoding a protein with dehydrocholesterol-delta7-reductase activity and/or dehydrocholesterol-delta24-reductase activity, wherein the nucleic acid(s) is or (respectively) are under control of a preferably constitutively active promoter for making an organism for a kit according to claim 10, wherein a precursor organism is transformed with the nucleic acid construct containing a nucleic acid encoding a membrane protein of a mammal, in particular a human membrane protein, preferably a plasma membrane protein, which is under control of a preferably constitutively active promoter, wherein the precursor organism preferably is a genetically modified organism with a reduced C24-methyltransferase and/or delta22-desaturase activity as compared to the wild-type.

12. The use according to claim 11, the nucleic acid construct further comprising a nucleic acid or a plurality of nucleic acids, identical or different, encoding a protein with HMG-CoA reductase and/or squalene epoxidase and/or lanosterol demethylase activity, wherein the nucleic acid(s) is or (respectively) are under control of a preferably constitutively active promoter.

13. A membrane extract, in particular a plasma membrane extract, obtainable from an organism according to one of claims 1 to 7 by disruption of the organism and separation of the membrane, in particular of the plasma membrane, comprising desmosterol and/or cholesterol and/or 7-dehydrocholesterol and/or lathosterol, as well as a membrane protein of a mammal, obtained from the gene encoding a membrane protein of a mammal.

## Revendications

1. Organisme vivant non mammifère génétiquement modifié isolé, ayant une activité de HMG-CoA réductase augmentée par rapport au type sauvage, et ayant une activité de C24-méthyltransférase et/ou de delta22-désaturase réduite par rapport au type sauvage,
**caractérisé en ce**
**que** l'organisme a une activité de déhydrocholestérol-delta7-réductase augmentée par rapport au type sauvage, et
**que** l'organisme en outre est transformé avec un gène codant pour une protéine membranaire d'un mammifère, sous contrôle d'un promoteur.

2. Organisme selon la revendication 1, **caractérisé en ce que** l'organisme en outre a une activité de déhydrocholestérol-delta24-réductase augmentée par rapport au type sauvage.

3. Organisme selon une des revendications 1 ou 2, **caractérisé en ce que** l'organisme a une activité de squalène époxidase et/ou lanostérol déméthylase augmentée par rapport au type sauvage.

4. Organisme selon une des revendications 1 à 3, **caractérisé en ce que** l'organisme est un organisme procaryotique, en particulier choisi à partir du groupe consistant en des bactéries des genres Bacillus, Escherichia, Lactobacillus, Corynebacterium, Acetobacter, Acinetobacter et Pseudomonas ou un organisme eucaryotique, en particulier choisi à partir du groupe consistant en des algues, levures, champignons, cellules d'insecte, mousses et plantes.

5. Microorganisme selon la revendication 4, **caractérisé en ce que** la levure est choisie à partir du groupe consistant en Saccharomyces cerevisiae, Saccharomyces delbrückii, Saccharomyces italicus, Saccharomyces ellipsoideus, Saccharomyces fermentati, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum et Saccharomycodes ludwigii, aussi bien que des levures du genre Kluyveromyces comme K. lactis K. marxianus var. marxianus, K. thermotolerans, aussi bien que des levures du genre Candida comme Candida utilis, Candida tropicalis, Candida albicans, Candida lipolytica et Candida versatilis, aussi bien que des levures du genre Pichia comme Pichia stipidis, Piachia pastoris et Pichia sorbitophila, aussi bien que des levures des genres Cryptococcus, Debaromyces, Hansenula, Saccharomycecopsis, Saccharomycodes, Schizosaccharomyces, Wickerhamia, Debayomyces, Hanseniaspora, Kloeckera, Zygosaccharomyces, Ogataea, Kuraishia, Komagataella, Metschnikowia, Williopsis, Nakazawaea, Cryptococcus, Torulaspora, Bullera, Rhodotorula, Willopsis et Sporobolomyces.

6. Organisme selon une des revendications 1 à 5, **caractérisé en ce que** l'organisme, en particulier la membrane, en particulier la membrane de plasma, de l'organisme a un contenu augmenté de désmostérol et/ou cholestérol et/ou 7-déhydrocholestérol et/ou lathostérol par rapport au type sauvage.

7. Organisme selon une des revendications 1 à 6, **caractérisé en ce que** le gène codant pour une protéine membranaire d'un mammifère est un gène codant pour une protéine membranaire humaine, de préférence une protéine membranaire de plasma, et que ce gène est sous contrôle d'un promoteur constitutivement actif.

8. Utilisation d'un organisme selon une des revendications 1 à 7 pour préparer une protéine membranaire d'un mammifère, en particulier une protéine membranaire humaine, de préférence une protéine membranaire de plasma, **caractérisée en ce que** l'organisme transformé est cultivé, de préférence sans addition de cholestérol et/ou désmostérol, et qu'à l'expiration d'une durée de culture prédéterminée la protéine membranaire est isolée d'un surnagé de culture et/ou de l'organisme.

9. Utilisation d'un organisme selon une des revendications 1 à 7 pour le dépistage de substances liant à une protéine membranaire d'un mammifère, en particulier à une protéine membranaire humaine, de préférence à une protéine membranaire de plasma, **caractérisée en ce que** l'organisme ou un extrait membranaire de l'organisme, après une culture de l'organisme précédente, est contacté avec une substance prédéterminée ou un mélange de substances prédéterminées, que des modifications ou l'absence de modifications de propriétés de l'organisme, de l'extrait membranaire et/ou le liage ou le non liage d'une substance à la protéine membranaire est mesuré, et que, si des modifications et/ou un liage sont mesurés, la substance ou le mélange de substances est classé comme liant à la protéine membranaire.

10. Kit pour la réalisation d'un procédé selon la revendication 9, avec un organisme vivant non mammifère génétiquement modifié, ayant une activité de HMG-CoA réductase augmentée par rapport au type sauvage, ayant une activité de C24-méthyl-transférase et/ou de delta22-désaturase réduite par rapport au type sauvage, et ayant une augmentée activité de déhydrocholestérol-delta7-réductase par rapport au type sauvage, avec une construction d'acide nucléique apte à la transformation de l'organisme et comportant un acide nucléique codant pour une protéine membranaire d'un mammifère, en particulier une protéine membranaire humaine, de préférence une protéine membranaire de plasma, qui est sous contrôle d'un promoteur de préférence constitutivement actif, et avec un mode d'emploi pour transformer l'organisme avec la construction d'acide nucléique, pour cultiver l'organisme avant et après la transformation, pour contacter l'organisme avec une substance prédéterminée ou un mélange prédéterminé de substances, et pour mesurer une modification d'une propriété de l'organisme et/ou d'un liage de la substance ou du mélange de substances à la protéine membranaire.

11. Utilisation d'une construction d'acide nucléique, en particulier d'un plasmide ou d'un shuttle vector, comprenant un acide nucléique ou plusieurs acides nucléiques, identiques ou différents, codant pour une protéine avec activité de déhydrocholestérol-delta7-réductase et/ou activité de déhydrocholestérol-delta24-réductase, dans laquelle le (les) acide(s) nucléique(s) est ou (respectivement) sont sous contrôle d'un promoteur de préférence constitutivement actif pour préparer un organisme pour un kit selon la revendication 10, dans laquelle un organisme précurseur est transformé avec la construction d'acide nucléique comportant un acide nucléique codant pour une protéine membranaire d'un mammifère, en particulier une protéine membranaire humaine, de préférence une protéine membranaire de plasma, qui est sous contrôle d'un promoteur de préférence constitutivement actif, dans laquelle l'organisme précurseur de préférence est un organisme génétiquement modifié ayant une activité de C24-méthyltransférase et/ou de delta22-désaturase réduite par rapport au type sauvage.

12. Utilisation selon la revendication 11, la construction d'acide nucléique en outre comprenant un acide nucléique ou plusieurs acides nucléiques, identiques ou différents, codant pour une protéine avec activité de HMG-CoA réductase et/ou squalène époxidase et/ou lanostérol déméthylase, dans laquelle le (les) acide(s) nucléique(s) est ou (respectivement) sont sous contrôle d'un promoteur de préférence constitutivement actif.

13. Extrait membranaire, en particulier extrait membranaire de plasma, obtenable à partir d'un organisme selon une des revendications 1 à 7 par disruption de l'organisme et séparation de la membrane, en particulier de la membrane de plasma, comprenant de désmostérol et/ou cholestérol et/ou 7-déhydrocholestérol et/ou lathostérol, aussi bien qu'une protéine membranaire d'un mammifère, obtenue à partir du gène codant pour une protéine membranaire d'un mammifère.
